Europäisches Patentamt

**European Patent Office**

Office européen des brevets

(19)

(11) Publication number: **0 052 002**
**A2**

(12) # EUROPEAN PATENT APPLICATION

(21) Application number: **81305308.9**

(22) Date of filing: **09.11.81**

(51) Int. Cl.³: **C 12 N 15/00,** C 12 N 1/20,
C 12 P 21/02
// C12R1/19

(30) Priority: **10.11.80 GB 8036080**

(43) Date of publication of application: **19.05.82**
**Bulletin 82/20**

(84) Designated Contracting States: **BE CH DE FR GB IT LI
NL SE**

(71) Applicant: **G.D. Searle & Co., Box 1045, Skokie,
Illinois 60076 (US)**

(72) Inventor: **Tacon, William Charles Albert, 62 Whitelands
Road, High Wycombe Buckinghamshire (GB)**
Inventor: **Carey, Norman Henry, Russells Close High
Street, Chinnor Oxfordshire (GB)**
Inventor: **Emtage, John Spencer, 12 Benjamin House
Amersham Hill, High Wycombe Buckinghamshire (GB)**
Inventor: **Houghton, Michael, 63 Mill End Road, High
Wycombe Buckinghamshire (GB)**
Inventor: **Stewart, Andrew George, 4 Amersham Road,
High Wycombe Buckinghamshire (GB)**

(74) Representative: **Smith, Sydney et al, Elkington and Fife
High Holborn House 52/54 High Holborn, London
WC1V 6SH (GB)**

(54) Plasmid vectors, production and use thereof.

(57)  A plasmid having an insertion site for a DNA fragment adjacent to a bacterial promoter, the attenuator of which has been removed, and downstream from a prokaryotic ribosome binding site and optionally an initiator codon such that an inserted DNA fragment is under bacterial promoter control is disclosed.

The production and use of such plasmids is also disclosed.

This invention relates to plasmid vectors and to the production and use thereof; more particularly, it relates to trp attenuator (trp a) - minus expression plasmids designed to receive and express DNA fragments.

In general terms, it is a disadvantage of known vectors that they provide expression to a lesser degree than is desired. This general problem is at least partially solved by the present invention.

The five genes encoding the enzyme of the E. coli tryptophan biosynthetic pathway form the tryptophan operon and are negatively controlled by the unlinked regulatory gene trp R. The trp R$^+$ gene codes for a protein, the trp aporepressor, (see, for example, Zubay, G., et al, (1972), Proc. Natl. Acad. Sci. U.S.A., 69, 1100 - 1103). This protein, when complexed with L - tryptophan, binds to the trp operator sequence within the promoter region of the trp operon and represses transcription initiation by physically excluding the RNA polymerase from the region of the promoter where attachment and initiation of transcription occurs. When the trp aporepressor is not activated by tryptophan, it cannot bind to the operator. Thus, tryptophan production is adjusted to the requirements of the cell.

As well as regulation by repressor/operator interactions, transcription of the structural genes is controlled from a region of the operon termed the "attenuator", which is prior to the first structural gene of the biosynthetic pathway. RNA polymerase molecules that have initiated transcription at the promoter may either terminate transcription at the attenuator, in the 160 bp leader region of the operon, or continue transcription into the structural genes. Termination of transcription at trp a is regulated and varies in response to changes in the levels of charged v uncharged tRNA trp. The critical factor influencing attentuation is translation of the trp leader region. The trp operon produces a leader polypeptide of 14 amino acids that contains the sequence trp - trp - arg at positions 10, 11 and 12. The presence in this leader peptide of the amino acid that is the end product of the operon being regulated and the knowledge that transcription termination varies in response to changes in the levels of charged or uncharged tRNA specific for tryptophan suggests a role for the leader peptide in regulation of termination. This role allows the cell to monitor the availability of tryptophan; thus, synthesis of the entire leader peptide would reflect a bountiful supply of tryptophan and therefore most transcription would be terminated at the attentuator, on the other hand, if tryptophan was scarce, synthesis of the leader peptide could not be completed and transcription would not be terminated at the attentuator, but would continue into the structural genes leading to the synthesis of tryptophan.

More particularly, under conditions of high cellular tryptophan, approximately 85% of the RNA polymerase molecules which initiate transcription at the trp promoter proceed only as far as an AT-rich region situated 134-152 nucleotides into the 162 bp trp leader, (see, for example, Bertrand, K., et al, (1976), J. Mol. Biol., 103, 319 - 337). These transcripts terminate at one of five positions within a run of 8 adenine nucleotides to give RNA's of 137-141 nucleotides in length, (see, for example, Bertrand, K., et al, (1977), J. Mol. Biol., 117, 227 - 247). In molar terms, this represents an 8 - 10 fold excess of 'leader' RNA relative to structural gene RNA, (see Bertrand, et al, (1976), loc cit). Upon starving cells of tryptophan, transcription termination at the attenuator site is relieved, (see, for example, Bertrand, K., and Yanofsky, C., (1976), J. Mol. Biol., 103, 339 - 349) and RNA polymerase proceeds into the structural genes. Although tryptophan seemingly appears to be the effector in the attenuation response, it is actually the level of charged versus uncharged tRNA$^{Trp}$ which is being monitored by the cell, (see, for example, Morse, P.E., and Morse, A.N., (1976), J. Mol. Biol., 103, 209 - 226).

As mentioned above, a characteristic of amino acid attenuator regions is the presence within the region of a sequence coding for a small peptide incorporating in series at least two amino acids which are the end product of the operon. In the trp leader a sequence coding for a

0052002

14 amino acid peptide incorporating tandem tryptophan codons in the tenth and eleventh amino acid positions is situated 27 bp from the startpoint of transcription. Furthermore, the sequence is preceded by a SD site as efficient in binding ribosomes as the trpE SD site, (see, for example, Platt, T., et al, (1976), J. Mol. Biol., 103, 411 - 420), which would suggest its probable translation in vivo. It has been proposed (see, for example Zurawski, G., et al, (1978), Proc. Natl. Acad. Sci. U.S.A., 75, 5988 - 5992), that the presence within the attenuator or leader peptide of tandem tryptophan codons provides a means whereby the level of cellular tryptophan may be monitored. Thus, translation of this sequence in full would indicate an abundance of charged tRNA$^{Trp}$ and result in transcription termination at the attenuator. Alternatively, starvation of tryptophan would only allow translation up to the tandem codons, this event resulting in transcription continuing into the structural genes. However, although translation of the entire peptide is indeed a prerequisite for termination, the precise signal for the leader RNA to be released from the RNA polymerase - DNA template complex is the formation within the leader RNA of a predetermined stem and loop structure prior to the point of termination.

Although the presence of the leader peptide has not been detected in vivo, indirect evidence that it is indeed synthesised has been provided, (see, for example, Miozzari, G.F., and Yanofsky, C., (1978),
J. Bacteriol., 133, 1457 - 1466; and Sommer, H., et al
(1978), J. Mol. Biol., 123, 457 - 469).  There were
detected hybrid protein fusions synthesised as a result
of joining part of the leader peptide coding sequence
in-phase with other bacterial genes, the fusions containing
the leader peptide sequence as the N-terminal portion.

Four regions within the trp leader RNA may
potentially form three stable stem and loop structures,
(see for example, Oxender, D.J., et al, (1979), Proc.
Natl. Acad. Sci. U.S.A., 76, 5524 - 5528).  These regions
are:  region 1 from nucleotide positions 52 to 68
(incorporating the tandem tryptophan codons), region 2
from positions 74 to 94, region 3 from positions 108 to
121 and region 4 from positions 126 to 134.

The potential stem and loops are formed on
base pairing between nucleotides within regions 1 and
2 (stem and loop 1:2), 2 and 3 (stem and loop 2:3) and 3
and 4 (stem and loop 3:4); the formation of the 3:4 stem
and loop signalling termination.  A model has been
proposed, (see Oxender, et al, loc cit), for transcription
termination at the attenuator based on these stem and loop
structures.

Under conditions of tryptophan excess, the
ribosome translating the attenuator peptide sequence
covers regions 1 and 2 of the newly transcribed RNA,
thus allowing regions 3 and 4 to base pair which results in termination.  When tryptophan is limiting, the ribosome stalls at the two tryptophan codons in region 1, but does not mask region 2 which base pairs with region 3 on its transcription, the formation of the 2:3 loop excluding the 3:4 loop.  Transcription by RNA polymerase therefore continues into the structural genes.  If, however, the attenuator peptide sequence is not translated (e.g. in vitro transcription reactions using DNA fragments) regions 1 and 2 base pair on the immediate transcription thereof so-excluding the formation of the 2:3 loop, this in turn allowing the formation of the 3:4 loop which terminates transcription.  This latter situation therefore accounts for the rather high degree of termination observed in vitro where 95% of the initiating RNA polymerase molecules terminate at the attenuator, (see, for example, Lee. F., and Yanofsky, C., (1977), Proc. Nat. Acad. Sci. U.S.A., 74, 4365 - 4369).

A consequence of attenuation is that complete derepression is never achieved naturally as even under tryptophan starvation conditions sufficient of the amino acid must be synthesised to allow completion of the attenuator peptide in some instances.  Complete induction, however, may be achieved by using 3-β-indole acrylic acid (IAA).  IAA has three effects on cells:

(i) It competes  with tryptophan for the repressor protein to which it binds with great affinity, (see, for example, Rose, J.K., et al, (1973), Nature New Biol., 245, 133 - 137). Moreover, as the IAA/repressor complex cannot bind to the operator, the cells are effectively rendered trp R⁻.

(ii) It inhibits the charging of tryptophan by tryptophanyl - tRNA synthetase and thereby overcomes attenuation, (see, for example Doolittle, W.F., and Yanofsky, C., (1968), J. Bact. 95, 1283 - 1294).

(iii) It inhibits tryptophan formation and starves cells of this amino acid (see, Doolittle and Yanofsky, loc cit).

While a combination of (i) and (ii) leads to complete derepression of transcription, a prerequisite for efficient gene expression, the fact that the cell is simultaneously starved of tryptophan results in these efficiencies not being obtained, especially if the gene product of interest is rich in tryptophan.

If, however, the attenuator region is removed, transcriptional control would be solely at the repressor level and could be maintained by the presence or absence of tryptophan.

Alternatively, the plasmid could be grown in trp R⁻ cells where expression would be constitutive.

A number of E. coli W3110 derivatives have been constructed, (see, Bertrand, et al, (1976), loc cit), from which the trp attenuator had been deleted, two of which (trpΔLE1417 and trpΔLE1413) were deleted from a point within the attenuator peptide sequence (22 to 25 bp from the N-terminus) to separate points within trp E, approximately 720 and 1200 bp of DNA having been deleted, respectively. These derivatives both produced elevated levels of downstream trp operon enzymes and corresponding mRNA. In a trp R$^-$ background an increase in trp B protein and distal mRNA of 8 - 10 fold was found compared with the levels in otherwise isogenic attenuator$^+$ strains (see Bertrand, et al, (1976), loc cit; and Miozzari and Yanofsky, loc cit). A similar type of mutant strain where the trp attenuator was deleted on fusing the lac I repressor gene to the attenuator peptide sequence, (21 bp from the N-terminus) produced 1% of the soluble protein as a hybrid lac repressor in a trp R$^-$ strain, (see Sommer, et al, loc cit). On DNA sequencing through this region, it was revealed that an in-phase fusion had been created incorporating the attenuator peptide sequence, 28 bp of lac I leader and the lac I gene.

These two examples of attenuator deletion illustrate some advantage in removing this region when an increase in downstream expression is desired. Based simply on the level of expression achieved for a single lac I gene fusion, an increase in gene number to 60 should in theory result in 60% of the soluble protein as repressor. In practice, this value will be more in the region of 30%. Considerable advantage may however be gained on expressing eukaryotic genes in attenuator minus plasmids, as the resulting increase in mRNA would to some extent compensate for translational inefficiencies.

The two trp DNA regions containing the deletions trpΛLE1417 and trpΔLE1413 were transferred onto a λ trp transducing phage and the ColEI type cloning vehicle, pVH51, (see Miozzari and Yanofsky, loc cit). These were then used to examine trp operon expression in trp R⁻ and trp R⁺ backgrounds, both in vitro and in vivo. E. coli cells separately infected with the two attenuator minus phages, λtrpED10ΔLE1413 and λtrpED10ΔLE1417 produced new trp proteins of 19Kd and 36Kd, respectively, the sizes of these proteins corresponding to fusions between the attenuator peptide N-terminus and the remainder of the trp E protein; the levels of the two proteins increasing on derepression. These same two proteins were produced by the attenuator minus trp plasmids in an in vitro transcription-translation system. Tryptic and chymotryptic digests performed on the two proteins released fragments which, when compared with those produced by trp E protein, confirmed the former as translation products resulting from in-phase fusions between the attenuator peptide sequence and the trp E gene.

The formation of stable fusions with the attenuator peptide as just described, (see Miozzari and Yanofsky, loc cit; and Sommer, et al, loc cit) suggests that the 14 amino acid peptide is synthesised in vivo.

Attempts to detect a protein of this size under trp control, (see Miozzari and Yanofsky, loc cit) were however unsuccessful. Nevertheless, as fusions with this peptide are stable, it provides a useful source for a fusion N-terminus incorporated in an attenuator-deleted trp expression plasmid. Furthermore, the ability of the attenuator peptide SD site to initiate translation implies that a gene suitably placed downstream of it (the attenuator peptide sequence having been removed) would probably be translated, as has been demonstrated for the lac Z SD site, (see, for example, Goeddel, D.V. et al, (1979), Nature, 281, 544 - 548).

Reference may also be made to published European Patent Application No. 0036776.

Certain novel plasmid vectors have now been constructed which surprisingly offer considerable advantages, inter alia in terms of expression.

In a first embodiment, the present invention provides a plasmid having an insertion site for a DNA fragment, e.g. a eukaryotic DNA fragment, adjacent to a bacterial promoter, in particular a trp-promoter, the attenuator of which has been removed, and downstream from a prokaryotic ribosome binding site and optionally an initiator codon such that an inserted DNA fragment is under bacterial promoter control.

Such a plasmid may be produced by a process which comprises cloning a bacterial promoter, the

-12-  0052002

attenuator of which has been removed, into an isolated plasmid, determining the position of an intended insertion site adjacent to and downstream from the promoter and providing an insertion site if it is not present.

A further embodiment of the present invention is directed accordingly.

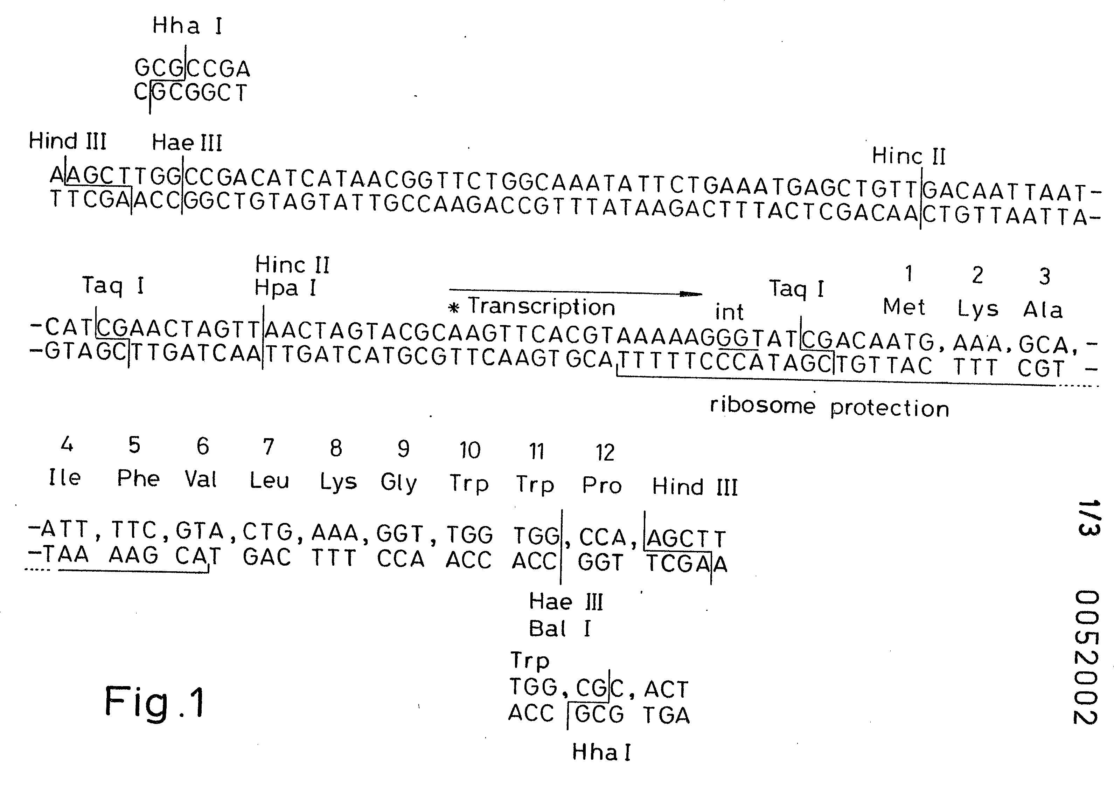

In arriving at the present invention, a sequence which was of particular interest and was studied is illustrated in Figure 1 of the accompanying drawings.

An examination of the illustrated trp DNA sequence reveals that a region bounded by the two Hha I sites located at +60 and -79 contains all of the necessary prerequisites for the controlling elements of an attenuator-minus expression plasmid of the fusion type. The 139 bp fragment contains:

(i) The site of interaction of RNA polymerase with the promoter (-59 to +18). In vitro transcriptional assays using fragments from the trp promoter region indicate that the left-ward boundary for promoter activity, upstream from the Hpa I site (see accompanying Figure 1), lies between positions -39 and -59 of the nucleotide sequence, (see, for example, Brown K.D., et al, (1978), J. Mol. Biol., 121, 153 - 177).

(ii) The operator region which interacts with the repressor. This overlaps the promoter region and is situated between positions -21 and -2.

(iii) The initiator codon for the attenuator peptide at positions +27 to +29 and coding sequence for a further 10 amino acids.

It was therefore decided to use such a fragment as a basis for the construction of plasmids of the above type. The cloning strategy involved repair of the Hha I ends with DNA polymerase I, attachment of Hind III linkers and insertion of the fragment into the Hind III site of pAT 153, (see, for example Twigg, A.J., and Sherratt, D., (1980), Nature, 283, 216 - 218).

For example, such a construction procedure in accordance with the present invention may comprise:

(a) restricting a plasmid of the so-called "pWT series", (see, for example, Tacon, W.C.A, et al, (1980), Molec. Gen. Genet., 177, 427), such as pWT 111 or pWT 221, using Hha I (EC 3.1.23.19);

(b) isolating the trp-promoter-containing Hha I fragment from an acrylamide gel;

(c) incubating the resulting fragment with DNA polymerase I (EC 2.7.7.7) to produce blunt-ended molecules;

(d) adding Hind III linkers and restricting using Hind III (EC 3.1.23.21);

(e) separating the fragment from unligated linkers on a gel and isolating the ~140 bp fragment; and

(f) cloning this into the Hind III site of pAT 153.

Such a procedure produces non-mobilisable (nic⁻) expression plasmids, designated pWT 501 or pWT 502, depending on the orientation of the trp promoter.

The Hha I fragment may also be used as a source of DNA in the construction of plasmids suitable for the expression of gene sequences from the N-termini thereof. Present within this fragment is a Taq I site (+22) located immediately downstream of the attenuator peptide SD site, which on conversion to a suitable cloning-expression site, e.g. Hind III, allows the insertion of foreign DNA coding for a polypeptide and its subsequent expression as mature or pre-polypeptide. Such will be discussed in more detail below.

The plasmid designated pWT 501 has a molecular length of 3805 bp; a Pst I site; an EcoRI site 752 bp from the Pst I site; a first Hind III site 31 bp from the EcoRI site; a Hpa I site 72 bp from the first Hind III site; a second Hind III site 75 bp from the Hpa I site; a Bam HI site 346 bp from the second Hind III site; a Sal I site 275 bp from the Bam HI site; and the Pst I site 2254 bp from the Sal I site.

This constitutes one embodiment of the present invention.

The plasmid designated pWT 502 has a molecular length of 3805 bp; a Pst I site; an EcoRI site 752 bp from the Pst I site; a first Hind III site 31 bp from the EcoRI site; a Hpa I site 75 bp from the first Hind III site; a second Hind III site 72 bp from the Hpa I site;

a Bam HI site 346 bp from the second Hind III site; a Sal I site 275 bp from the Bam HI site; and the Pst I site 2254 bp from the Sal I site.

This constitutes another embodiment of the present invention.

pWT 501 may be produced by a process which comprises restricting pWT 221 using Hha I, isolating the trp-promoter-containing fragment, incubating this fragment with DNA polymerase I, ligating the resulting blunt-ended fragment to Hind III linkers, restricting the ligated material using Hind III, isolating the trp-promoter-containing fragment, ligating this fragment to Hind III-cut, bacterial alkaline phosphatase (EC 3.1.3.1)-treated pAT 153, transforming E. coli K 12 HB101, (genotype gal$^-$, lac$^-$, ara$^-$, pro$^-$, leu, str$^r$, rec A$^-$, r$_k^-$, M$_k^-$; see, for example Boyer, H.W., and Roullard-Dussoix, D., J. Mol. Biol., 41, 459 - 472), using the resulting plasmid and selecting for ampicillin-resistance and tetracycline-resistance.

pWT 502 may be produced by a similar process, except that one selects for ampicillin-resistance and tetracycline-sensitivity.

Further embodiments of the present invention are directed accordingly.

For purposes of exemplification, the following illustrates the construction of attenuator minus trp expression plasmids, specifically pWT 501 and pWT 502, based on fusion into the attenuator peptide.

As a source of the 139 bp Hha I <u>trp</u> promoter fragment any one of the pWT 1X1 or pWT 2X1 series plasmids could be used. However, the pWT 1X1 series plasmids, on restriction with Hha I also produce a 141 bp fragment of pBR 322, (see, for example, Bolivar, F., <u>et</u> <u>al</u>, (1977), Gene, <u>2</u>, 95 - 113), origin (located in the Hae II B fragment) which co-migrates with the <u>trp</u> promoter fragment. For this reason, it was decided to use nic⁻ pWT 2X1 series plasmid (pWT 221).

pWT 221 (5 µg) was restricted using Hha I, the DNA electrophoresed on a 5% w/v acrylamide gel and the 139 bp band excised and recovered from the acrylamide by a conventional method, (see, for example, Hindley, J., and Phear, G.A. (1979), Nucleic Acids Res., $\underline{7}$, 361 - 375). More particularly, 0.5 ml of acrylamide gel extraction buffer (AGEB), (0.5 M $NH_4OAc$, 10 mM $Mg(OAc)_2$, 0.1% w/v sodium dodecyl sulphate (SDS), 0.1 mM ethylene diamine tetra-acetic acid (EDTA)) was added to the crushed gel slice in an Eppendorf centrifuge tube and the mixture shaken gently for 16 hours at 37°C. This allowed the DNA to diffuse into the buffer and, depending on size, normally resulted in recoveries of >50%. (DNA's greater than 2 Kb gave poorer recoveries.) The acrylamide was then removed by centrifugation through a Whatman 52 filter paper plug placed at the bottom of a 2 ml disposable syringe.

The filtrate was then diluted four-fold with water and passed through a 1 ml DE52 column pre-washed with 1/5 AGEB whereupon the DNA absorbed. The column was further washed with 1/5 AGEB and the DNA then eluted with 1.5 ml of 1.1 M NaCl in 1/5 AGEB, collecting the final 1 ml fraction. Ethidium bromide was removed by two extractions with n-butanol and the DNA precipitated by adding 2.5 volumes of ethanol.

The 139 bp fragment migrates to a position immediately below a trimer band containing fragments of 151, 152 and 153 bp. It might therefore be expected that a small percentage of these larger fragments would be extracted along with the trp fragment.

To enable Hind·III linkers to be attached, the Hha I ends were first removed using DNA polymerase I according to a known method (see, for example, Seeburg, P.H., et al, (1978), Nature, 276, 795 - 798). In order to remove the 3' extension, from 0.5 to 3.5 μg of DNA was incubated in 50 mM Tris-HCl (tris(hydroxymethyl)aminomethane hydrochloride), pH 7.8, 10 mM $MgCl_2$, 1 mM 2-mercaptoethanol, in a volume of from 10 to 50 μl using from 0.25 to 1.5 units (1 unit is the amount that incorporates 10 n moles of total nucleotides into an acid-precipitable fraction in 30 minutes at 37°C using poly-d(A-T) as primer according to Richardson, C.C., et al, (1964), J. Biol. Chem., 239, 222) of DNA polymerase I. The mixture was incubated for 20 minutes at 10°C, after which an aliquot could be used directly for blunt end ligation or the whole extracted with equal volumes of phenol and chloroform, and the DNA precipitated with ethanol.

The blunt-ended fragment was then ligated to phosphorylated Hind III linkers, (5'-C C A A G C T T G G - 3', Collaborative Research, Waltham, Mass., U.S.A.), using a ratio of 50 moles of linker per mole of fragment. Hind III ends were produced by restricting the ligated material using Hind III and the whole was electrophoresed on a second 5% w/v acrylamide gel from which the trp-promoter band was recovered.

The ligation was conducted in known manner (see, for example Suigino, A., et al, (1977), J. Biol. Chem., 252, 3987 - 3994).

Ligations were performed in volumes of from 10 to 20 μl in 50 mM Tris-HCl, pH 7.8, 10 mM MgCl$_2$ 20 mM dithiothreitol (DTT), 1 mM ATP using from 0.2 to 1.0 units (1 unit is the amount that catalyses the conversion of 1 n mole of $^{32}P$ P$_i$ into (α/β $^{32}P$)-ATP in 20 minutes at 37°C according to Weiss, B., et al, (1968), J. Biol. Chem., 243, 4543) of T4 DNA ligase (EC 6.5.1.1) and from 0.1 to 2 μg of DNA. Reactions were incubated from 4 to 16 hours at 25°C.

Regarding recovery, the DNA was ethanol precipitated, resuspended in 20 μl of TE buffer and electrophoresed on a 5% w/v acrylamide gel. The linkered fragment was then extracted from the gel using the above-described conventional DEAE (diethylaminoethyl)-cellulose procedure.

This latter step removes unbound Hind III linker DNA which would otherwise interfere in the ligation to pAT 153. The trp fragment was then ligated to Hind III-cut, bacterial alkaline phosphatase-treated pAT 153 (0.1 µg) and the ligated molecules transformed into E. coli K 12 HB101 applying a nutrient agar-carbenicillin (100 µg/ml) selection.

As a precaution, tryptophan (100 µg/ml) was also added to the transformation plates to minimise trp controlled expression (in the absence of the attenuator, over-production of the 34kd tetracycline resistance protein could prove lethal). A total of 373 transformants was obtained.

To determine the probable orientation of the inserted trp fragment, transformants were screened for resistance to tetracycline, resistance identifying inserts where the trp promoter is transcribing into the tetracycline gene ('R' insertion). 100 transformants were streaked onto two sets of M9-casamino-acids minimal agar (100 ml M9 salts (10 x M9 salts is 60 g $Na_2HPO_4$, 30 g $KH_2PO_4$, 5 g NaCl and 10 g $NH_4Cl$ in one litre of distilled water), 5 g casamino acids (Difco), 2 g glucose, 0.25 g $MgSO_4.7H_2O$, 21.9 mg $CaCl_2.6H_2O$, 1 mg thiamine and 15 g agar in 1 litre of distilled water) plates both supplemented with carbenicillin (Beecham; sodium salt of α-carboxy benzylpenicillin) and tetracycline (10 μg/ml), but with one set additionally supplemented with tryptophan (100 μg/ml) for the reason mentioned above. Of the 100 transformants screened, 64 were $Ap^r Tc^r$ ± tryptophan and 36 $Ap^r Tc^s$ ± tryptophan, the latter resulting from the trp fragment inserting in the 'L' orientation or from pAT 153 fragments inserting in either orientation. Furthermore, the transformants grew equally well ± tryptophan, this signifying in the case of the 'R' inserts, that attenuator removal increases the frequency at which the tetracycline gene is transcribed under repressed conditions in comparison to pWT 101 which is unable to confer resistance to 10 μg/ml tetracycline when repressed, and, in both 'L' and 'R' inserts, that partial derepression is not lethal. It is interesting to note that almost twice as many $Tc^r$ transformants were obtained as opposed to $Tc^s$ transformants, implying some form of selection in favour of the former.

Restriction using Hind III of plasmid DNA prepared from both $Ap^r Tc^r$ and $Ap^r Tc^s$ transformants should produce fragments of 147 bp and 3658 bp, the former corresponding to the cloned trp promoter fragment and the latter to the linearised vector. It was found, however, that the size of the cloned fragment differed, a slightly larger fragment of 160 bp being produced by plasmid DNA isolated from $Ap^r Tc^s$ cells (a total of 8 $Tc^s$ transformants were screened for fragment size) while all the $Ap^rTc^r$ DNA's produced a fragment of the expected size, ⌣ 147 bp. Obviously, the $Ap^rTc^s$ plasmids must contain one of the larger pAT 153 fragments from pWT 221 and not the trp fragment. Assuming that few if any trp fragments have been cloned in the 'L' orientation, this would than account for the observed distribution of tetracycline resistance amongst the transformants, as the trp fragment should be relatively more abundant in the extracted DNA 'mixture' than the larger pAT 153 fragments. Rather than screen further $Ap^rTc^s$ transformants for the trp promoter fragment in the 'L' orientation, it was decided to reclone the 147 bp trp fragment into pAT 153 once it had been characterised and to isolate 'L' inserts from the resulting transformants.

Verification of the presence of the trp promoter in the probable 'R' inserts was provided by restriction using Hpa I which cuts the trp DNA only once at position -11 (see Figure 1 of the accompanying drawings) and has no site in pAT 153. The orientation of the trp promoter fragment was confirmed by double restriction with Bal I and Pst I. By virtue of the polymerase repair and ligation of Hind III linker DNA, a Bal I site is created at the Hind III junction downstream from the trp promoter (see Figure 1 of the accompanying drawings). Thus, for an 'R' insertion bands of 925 1421 and 1459 bp should be produced. Bal I and Pst I each cutting the vector molecule (pAT 153) once at positions 1416 and 2875, respectively, relative to the Hind III site (in a clockwise direction). As shown in a gel profile, the smallest band produced by an $Ap^r Tc^r$ plasmid previously shown to contain a Hpa I site is 925 bp, confirming the 'R' orientation. This plasmid was designated pWT 501.

Two reasons may be offered as to why an 'L' insertion was not isolated in the previous cloning; either that transcription from the trp promoter was interfering with transcription from the β-lactamase promoter such that no RNA was transcribed beyond the promoter (an unlikely event), or that excessive production of β-lactamase as a consequence of transcription from the trp promoter has a lethal effect. Either event is surprising considering that the trp promoter was repressed by tryptophan and that a similar plasmid, pWT 102 is stable at least in partially derepressing conditions (trp minus medium; stability has not been tested on IAA induction). Another attempt was therefore made to clone the promoter in the 'L' orientation.

0.8 μg of pWT 501 was restricted with Hind III and electrophoresed on a 5% w/v acrylamide gel from which the 147 bp fragment was excised and extracted. The purified fragment was then ligated to Hind III-cut pAT 153 and the ligated molecules transformed and plated. A total of 1442 transformants was obtained from which 100 were screened for resistance to tetracycline. Thus, 14 $Ap^r Tc^s$ transformants were obtained, the remainder all

being $Ap^r Tc^r$ (as the pAT 153 background represented only 2% of transformants obtained, the $Ap^r Tc^r$ transformants must have contained pWT 501; this however was not investigated further).

Hind III restriction of 8 plasmid DNAs prepared from the 14 $Ap^r Tc^s$ transformants produced both the vector molecule and the 147 bp *trp* fragment. Orientation of the fragment was again determined by double restriction with Bal I and Pst I. In the 'L' orientation bands of 783, 1459 and 1555 bp will be expected. The restriction profiles prove this to be the case for the two $Ap^r Tc^s$ plasmid isolates (in fact all 8 produce the same bands). It therefore appeared that 'L' insertions of the *trp* promoter fragment had been successfully isolated.

As a final check, all 8 'L' insert plasmids were restricted with Hae III and compared against similarly restricted pWT 501. Two Hae III sites are located in the 147 bp *trp* fragment, one at each end 5 bp from the Hind III site, both created by the polymerase repair reaction and subsequent attachment of Hind III linker. Thus, insertion of the 147 bp *trp* fragment in either orientation at the pAT 153 Hind III site which is located in a 191 bp Hae III fragment should result firstly, in the disappearance of the 191 bp fragment and secondly, in the production of 3 new Hae III fragments; fragments of 54 and 147 bp each containing a Hind III site and a 137 bp *trp* promoter fragment.  All 8 'L' insert plasmids produce the
expected Hae III fragments on restriction as does
pWT 501.  The production of these 3 Hae III fragments,
as well as confirming that the trp DNA is intact, also
confirms the predicted nucleotide sequence created by
the polymerase reactions and attachment of Hind III
linkers to the ends of the starting Hha I fragment
(it being shown above that the Hind III sites are
functional).  This therefore eliminated the need to
DNA sequence through these regions.  The 'L' orientated
plasmid was designated pWT 502.

The upstream (unwanted) Hind III site may be
removed in each case by conventional means, (see Tacon,
et al, loc cit), so producing plasmids designated pWT
511 and pWT 512.  Phase changing using exonuclease,
(see Tacon, et al, loc cit), produces pWT 521, 531 and
pWT 522, 532.

The plasmid designated pWT 511 has a molecular
length of 3809 bp; a Pst I site; an EcoRI site 752 bp
from the Pst I site; a Hpa I site 107 bp from the EcoRI
site; a Hind III site 75 bp from the Hpa I site; a Bam
HI site 346 bp from the Hind III site; a Sal I site
275 bp from the Bam HI site; and the Pst I site 2254 bp
from the Sal I site.

The plasmid designated pWT 512 has a molecular
length of 3809 bp; a Pst I site; an EcoRI site 752 bp
from the Pst I site; an Hind III site 31 bp from the EcoRI site; a Hpa I site 75 bp from the Hind III site; a Bam HI site 422 bp from the Hpa I site; a Sal I site 275 bp from the Bam HI site; and the Pst I site 2254 bp from the Sal I site.

The plasmid designated pWT 521 has a molecular length of less that 3809 bp; a Pst I site; an EcoRI site 752 bp from the Pst I site; a Hpa I site 107 bp from the EcoRI site; a Hind III site less than 75 bp from the Hpa I site; a Bam HI site 346 bp or less from the Hind III site; a Sal I site 275 bp from the Bam HI site; and the Pst I site 2254 bp from the Sal I site.

The plasmid designated pWT 531 has a molecular length of less than 3809 bp; a Pst I site; an EcoRI site 752 bp from the Pst I site; a Hpa I site 107 bp from the EcoRI site; a Hind III site less than 75 bp from the Hpa I site; a Bam HI site 346 bp or less from the Hind III site; a Sal I site 275 bp from the Bam HI site; and the Pst I site 2254 bp from the Sal I site.

The plasmid designated pWT 522 has a molecular length of less than 3809 bp; a Pst I site; an EcoRI site 752 bp from the Pst I site; a Hind III site 31 bp or less from the EcoRI site; a Hpa I site less than 75 bp from the Hind III site; a Bam HI site 422 bp from the Hpa I site; a Sal I site 275 bp from the Bam HI site; and the Pst I site 2254 bp from the Sal I site.

The plasmid designated pWT 532 has a molecular length of less than 3809 bp; a Pst I site; an EcoRI site 752 bp from the Pst I site; a Hind III site 31 bp or less from the EcoRI site; a Hpa I site less than 75 bp from the Hind III site; a Bam HI site 422 bp from the Hpa I site; a Sal I site 275 bp from the Bam HI site; and the Pst I site 2254 bp from the Sal I site.

These constitute further embodiments of the present invention.

pWT 511 may be produced by a process which comprises partially restricting pWT 501 using Hind III, isolating the linearised molecule, incubating this molecule with DNA polymerase I, ligating the blunt ends, transforming E. coli K 12 HB101 using the resulting plasmid, selecting for ampicillin-resistance and tetracycline-resistance and identifying rhe production by restriction enzyme analysis.

pWT 512 may be produced by a similar process using pWT 502 and selecting for ampicillin-resistance and tetracycline-sensitivity.

pWT 521 and pWT 531 may be produced by a process which comprises restricting pWT 511 using Hind III, digesting the exposed Hind III end(s) of the resulting linear molecule using exonuclease III (EC 3.1. 4.27) and S1 nuclease (EC 3.1.4.-), repairing the resulting staggered end(s) using DNA polymerase I, ligating to Hind III linkers, transforming E. coli K 12 HB101 using the resulting plasmid, selecting for ampicilline-resistance and tetracycline-resistance and identifying the phase-changed product.

pWT 522 and pWT 532 may be produced by a similar process using pWT 512 and selecting for ampicillin-resistance and tetracycline-sensitivity.

Such constitute further embodiments of the present invention.

As indicated above, the use of pWT 501 (or pWT 502) as an expression plasmid vehicles necessitates that the upstream Hind III site be removed to leave the Hind III site within the DNA coding for the attenuator peptide as the cloning site. This was accomplished by partial restriction of the plasmid using Hind III such that only one cut was produced.

More particularly, for purposes of exemplification, 2 µg of plasmid DNA was restricted for 60 minutes at 25°C in a reaction volume of 400 µl (10 mM Tris-HCl, pH 7.5, 10 mM $MgCl_2$, 1 mM DTT, 100 µg/ml gelatin and 50 mM NaCl) using 0.6 units (1 unit is the amount that digests 1 µg $\lambda$-DNA in 15 minutes at 37°C in 50 µl) of enzyme. The DNA was then extracted with equal volumes of phenol and chloroform and ethanol precipitated. Under these conditions the majority of plasmid molecules were cleaved only once. These linearised molecules were separated from uncut plasmid by means of preparative gel electrophoresis.

The DNA was then electrophoresed on a 3.5% w/v acrylamide gel, the linearised band was excised and recovered and the exposed Hind III ends removed by repairing using DNA polymerase I, followed by blunt end ligation. This procedure removes either of the two Hind III sites depending on which was exposed, the ligation mixtures should therefore be composed of both types in a 1:1 ratio. The ligated molecules were transformed into E. coli K 12 HB101 and plated on nutrient agar (25 g nutrient broth, 15 g agar in 1 litre of distilled water) supplemented with ampicillin (carbenicillin) and tryptophan (100 μg/ml). A total of 200 transformants was obtained. Plasmid DNAs were prepared from a number of transformants to test for the loss of the upstream Hind III site.

The removal of the upstream Hind III site was demonstrated by double restriction with Pst I (EC 3.1.23.31) and Hind III. Pst I cuts pWT 501 once, 783 bp from the upstream Hind III site. Thus, removal of the unwanted upstream Hind III site produces bands of 934 and 2875 bp on double restriction, while bands of 783 and 3026 bp are produced on removal of the downstream 'expression' site. Both types of plasmid have been isolated. The latter plasmid having had the upstream Hind III site removed was designated pWT 511, the phasing at the Hind III site of this plasmid being the same as for pWT 111 and pWT 121.

As indicated above, the derivative of pWT 501 is designated pWT 511.

0052002

An analogous procedure may be applied to the production of the corresponding derivative of pWT 502, pWT 512.

pWT 511, for example, being a fusion plasmid, is only capable of expressing gene sequences inserted at the Hind III site in a single phase, hence the need for the above-mentioned phase-changed derivatives.

The cloning of DNA into the Hind III site of pWT 511 or, indeed, pWT 512, where the phasing of the inserted DNA is in alignment with that of the attenuator peptide, will ultimately result in the synthesis of a fusion polypeptide containing an N-terminus of 12 amino acids, 11 derived from the attenuator peptide and 1 derived from the Hind III linker. In order to minimise and, indeed, reduce the number of N-terminal amino acids preceding the inserted amino acid sequence, phase-changing was accomplished by the removal of a number of nucleotides from the N-terminus. This was effected by a controlled exonuclease III, S1 nuclease digestion of the exposed Hind III ends, repair of staggered ends using DNA polymerase I, attachment of Hind III linker and recircularisation. The amount of DNA removed and the phasing through the Hind III site were determined by sequencing the Hind III region of plasmid DNA isolated from a number of bacterial colonies derived from transformations using the exonculease-treated DNA.

As indicated above, the present invention also relates to the construction of gene expression plasmids designed to allow direct translation of inserted gene sequences from the N-termini thereof.

A variety of procedures exist for the production in E. coli of polypeptides derived by means of recombinant DNA technology. These maybe categorised into three groups depending on the mode of synthesis thereof:

(i)    Fusion proteins. The desired protein is synthesised as part of a larger fusion product having an N-terminus composed of amino acids derived from a prokaryotic E. coli protein, (see, for example, Seeburg, P.H., et al, (1978), Nature, 276, 795).

(ii)   Cleavable fusion proteins. The protein is initially produced as a fusion product, but may be further processed by either chemical means, (see, for example, Itakura, K., et al, (1977), Science, 198, 1056 - 1063), or enzymatic means (see, for example, Shine, J., et al, (1980), Nature, 285, 456 - 461, or Tamladge, K., et al, (1980), Proc. Natl. Acad. Sci. U.S.A., 77, 3369 - 3373) to yield the mature product.

(iii)  Polypeptides synthesised from the N-termini thereof. In this case translation is initiated directly from the ATG or GTG of the inserted gene sequence by virtue of the placement thereof directly downstream of a ribosome binding site with no intervening bacterial initiation codon, (see, for example, Roberts, T.M., et al, (1979), Proc. Natl. Acad. Sci. U.S.A., 76, 5596 - 5600).

All three procedures clearly have advantages, for example, the fusion protein procedure might allow the production of a larger, but stable polypeptide where the native polypeptide might be unstable, e.g. susceptible to protease attack in vivo. However, the viability of large scale production of mature polypeptide by chemical or enzymatic cleavage of a fusion polypeptide would be governed by the extra cost that this processing would involve. The production of mature or pre-polypeptide in vivo would therefore seem a more feasible approach, provided that the product is stable. There is also the added advantage that only one expression plasmid need be constructed since, unlike the case of fusion products, phasing is no longer a problem.

The prerequisites for the construction of a plasmid capable of allowing the synthesis of mature or pre-polypeptide from an inserted gene sequence are:

(i) a ribosome binding site situated immediately upstream of a unique restriction endonuclease site suitable for cloning;

(ii) efficient transcription through the ribosome binding site and inserted sequence from a controllable promoter-operator region;

(iii) siting of (i) and (ii) on a suitable plasmid.

As mentioned above, these conditions may be satisfied by utilising a region from the tryptophan operon of E. coli, namely the section of DNA containing the tryptophan promoter-operator and ribosome binding site for the attenuator peptide.

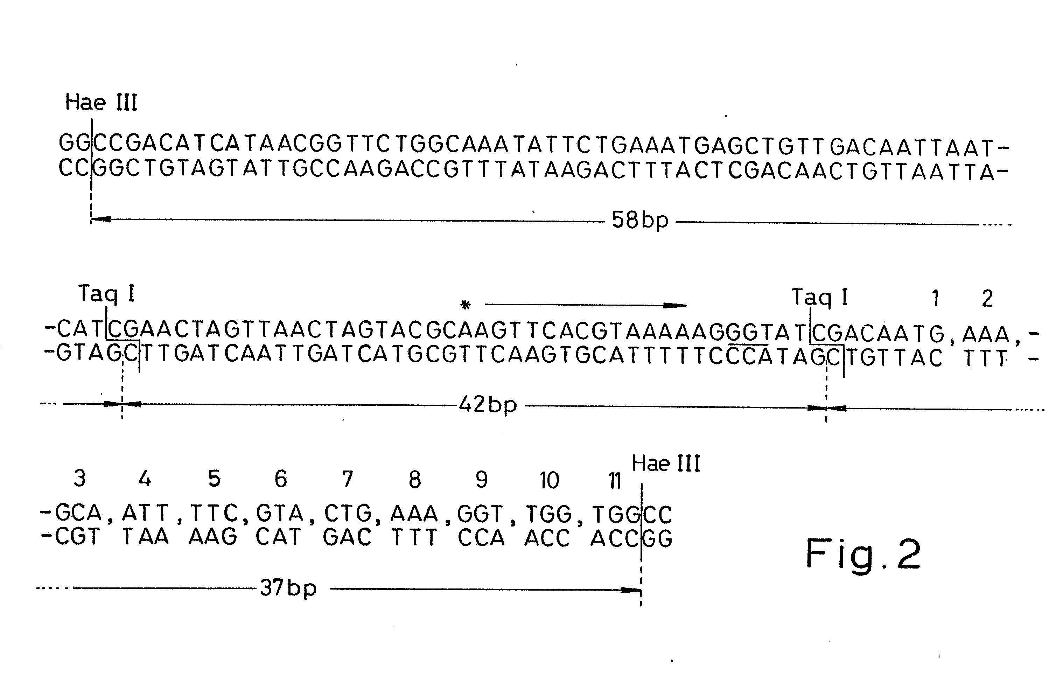

The above may be illustrated by reference to Figure 2 of the accompanying drawings relating to the isolation of the Hae III - Taq I promoter fragment and the cloning thereof in pAT 153:

The trp promoter-operator region and ribosome binding site for the attenuator peptide are situated on a 137 bp fragment  produced by cleaving, for example, pWT 501 using Hae III (see Figure 2 of the accompanying drawings).  The 100 bp fragment (-78 to +22) containing only the trp promoter-operator and ribosome binding site is isolated from this Hae III fragment by a partial digest using Taq I.  The exposed Taq I end of this fragment  is situated 2 nucleotides from the DNA sequence GGT which may potentially base-pair with a complementary region at the 3'-end of the 16S ribosomal RNA, (see, for example, Platt, T., (1978), The Operon, (Eds. Miller and Reznikoff), Cold Spring Harbor Laboratory, 263 - 302). Conversion of this site to a suitable cloning-expression site, e.g. Hind III, therefore allows the insertion of foreign DNA coding for a polypeptide and its subsequent expression as mature or pre-polypeptide.

For purposes of exemplification, the following illustrates the construction of pWT 550 and pWT 560 and hence pWT 551. pWT 550 and pWT 560 have two insertion sites and may be regarded as precursors of pWT 551, an attenuator minus trp expression plasmid which allows the synthesis of a native polypeptide from an inserted gene sequence.

20 ug of pWT 501 was restricted using Hae III and electrophoresed on a 5% w/v acrylamide gel. The 137 bp band was excised from the gel and the DNA recovered from the acrylamide. Removal of the unwanted 37 bp Taq I - Hae III fragments (containing the coding sequence for 11 amino acids of the attenuator peptide) was accomplished by partial restriction of the 137 bp Hae III fragment using Taq I and recovery from the acrylamide of the 100 bp band after electrophoresis of the partial products on a second 5% w/v acrylamide gel.

More particularly, the partial restriction using Taq I was carried out as follows:

0.42 µg of the 137 bp fragment was restricted for 12 minutes at 65°C in a reaction volume of 200 µl (10 mM Tris-HCl, pH 7.5, 10 mM MgCl$_2$, 1 mM DTT, 100 µg/ml gelatin, 5 mM NaCl) using 0.2 units (1 unit is the amount that digests 1.0 µg of $\lambda$ DNA in 15 minutes at 37°C in a total reaction mixture of 0.05 ml) of Taq I. The reaction was then terminated by the addition of 10 µl of 0.4 M EDTA and 4 µl of 10% w/v SDS, extracted with equal volumes of phenol and chloroform and ethanol precipitated.  The 100 bp DNA fragment produced by this partial restriction was separated from the other fragments in the mixture by preparative gel electrophoresis.

Prior to the ligation of Hind III linkers to this fragment, the exposed Taq I staggered end was repaired using DNA polymerase I.  The 5' extension was repaired by a conventioanl method (see, for example, Seeburg, P.H., et al, (1978), Nature, 276, 795 - 798). More particularly, from 0.5 to 3.5 µg of DNA was incubated in 50 mM Tris-HCl, pH 7.8, 10 mM $MgCl_2$, 1 mM 2-mercapto- ethanol, 0.1 mM each of dATP, dCTP, dGTP, dTTP in a volume of from 10 to 50 µl using from 0.25 to 1.5 units (1 unit is the amount that incorporates 10 n moles of total nucleotides into an acid-precipitable fraction in 30 minutes at 37°C using poly-d(A-T) as primer according to Richardson, C.C., et al, (1964), J. Biol. Chem., 239, 222) of DNA polymerase I.  The mixture was incubated for 20 minutes at 10°C, after which an aliquot could be used directly for blunt end ligation or the whole extracted with equal volumes of phenol and chloroform, and the DNA precipitated with ethanol. Linkers were attached in a ratio of 50 moles of linker per mole of fragment.  Hind III ends  were produced by restricting the ligated material using Hind III.

Unbound linker was removed from the ligated material by chromatography through a pre-equilibrated (50 mM NaCl, 0.1% w/v SDS) 50 x 0.7 cm column of G 150 "Sephadex", (Registered Trade Mark), (bead form, crosslinked dextran gel), the precipitated DNA having been re-suspended in 50 μl of 50 mM NaCl, 0.1% w/v SDS. The excluded peak containing the trp-promoter fragment and attached linkers was pooled and the DNA ethanol precipitated. This material was then ligated to Hind III-cut bacterial alkaline phosphatase-treated pAT 153 and the ligated molecules were transformed into E. coli K 12 HB101. Transformants were selected for on nutrient agar (25 g nutrient broth, 15 g agar in 1 litre of distilled water) supplemented with carbenicillin and tryptophan (100 μg/ml). A total of 85 transformants was obtained.

All transformants were screened for resistance to tetracycline by streaking on M9-casamino acids minimal agar supplemented with carbenicillin and tetracycline (10 μg/ml) ± tryptophan (100 μg/ml). 34 transformants were thus found to be $Ap^r Tc^r$ (probable 'R' orientated inserts) and 51 $Ap^r Tc^s$ (probable 'L' orientated inserts) both in the presence and absence of tryptophan. Plasmid DNAs were prepared from both 'L' and 'R' transformants.

The presence of cloned DNA was demonstrated by restricting plasmid DNA from both types of isolate with Hind III. Of the 4 'R' insert plasmids restricted, all produced a band of 111 bp, the size expected for the cloned _trp_ fragment (101 bp Hae III-Taq I fragment polymerase repaired plus 10 bp Hind III linker), while only 3 of the 12 'L' insert plasmids restricted produced a band of this size (1 in 4). The 9 remaining probable 'L' insert plasmids were all deletion derivatives (smaller in size that a true recombinant plasmid) and were not characterised further, indeed in view of this result it is impossible to state with certainty whether the deletants were of 'R' or 'L' inserted recombinants.

_Trp_ promoter orientation was determined by restriction with Hae III. As may be seen from accompanying Figure 2, a single Hae III site exists at the left-hand end of the 111 bp Hind III _trp_ fragment (-78). No such site is present at the right-hand end of the fragment prior to or after DNA polymerase I repair and attachment of a Hind III linker. Therefore, depending on the orientation of the _trp_ fragment (inserted at the Hind III site within the 191 bp Hae III fragment), fragments of 54 and 248 bp will be produced in addition to the expected pAT 153 Hae III bands for inserts transcribing towards the tetracycline gene ('R' orientation) and 147 and 155 bp for inserts transcribing away from the tetracycline gene ('L' orientation).

The Hae III digests confirmed the presence of the fragments as predicted. However, the 248 bp fragment produced by the 'R' insert plasmids appears to electrophorese abnormally as the size of this fragment is 258 bp when estimated from a migration profile. Cleavage of this fragment by Hpa I should produce fragments of 67 and 181 bp. It was found that the 67 bp band migrates to the predicted position while the 181 bp band is found as a dimer with a pAT 153 band of 184 bp.

Thus confirmed as true 'R' and 'L' insertions of the 111 bp Hind III trp fragment, the plasmids were designated pWT 550 and pWT 560, respectively.

To facilitate the use of pWT 550 as a Hind III cloning expression plasmid, the left-hand (upstream) Hind III site was removed as in the construction of pWT 511, by DNA polymerase I repair of partially Hind III restricted plasmid DNA (2.0 µg), followed by recircularisation. The ligated molecules were transformed into E. coli K 12 HB101 and transformants selected for on nutrient agar carbenicillin. A total of 199 transformants was obtained. Removal of the site was confirmed by restriction with Hind III and Pst I of plasmid DNAs prepared from a number of colonies. Bands of 783 and 2990 bp (111 + 4 + 2875 bp) are produced on removal of the downstream 'expression' site and 898 (783 + 4 + 111 bp) and 2875 bp on removal of the upstream site. The plasmid isolate having the upstream Hind III site removed was designated pWT 551.

The plasmid designated pWT 550 has a molecular length of 3769 bp; a Pst I site; an EcoRI site 752 bp from the Pst I site; a first Hind III site 31 bp from the EcoRI site; a Hpa I site 72 bp from the first Hind III site; a second Hind III site 39 bp from the Hpa I site; a Bam HI site 346 bp from the second Hind III site; a Sal I site 275 bp from the Bam HI site; and the Pst I site 2254 bp from the Sal I site.

The plasmid designated pWT 560 has a molecular length of 3769 bp; a Pst I site; an EcoRI site 752 bp from the Pst I site; a first Hind III site 31 bp from the EcoRI site; a Hpa I site 39 bp from the first Hind III site; a second Hind III site 72 bp from the Hpa I site; a Bam HI site 346 bp from the second Hind III site; a Sal I site 275 bp from the Bam HI site; and the Pst I site 2254 bp from the Sal I site.

The plasmid designated pWT 551 has a molecular length of 3773 bp; a Pst I site; an EcoRI site 752 bp from the Pst I site; a Hpa I site 107 bp from the EcoRI site; a Hind III site 39 bp from the Hpa I site; a Bam HI site 346 bp from the Hind III site; a Sal I site 275 bp from the Bam HI site; and the Pst I site 2254 bp from the Sal I site.

The plasmid designated pWT 561 has a molecular length of 3773 bp; a Pst I site; an EcoRI site 752 bp from the Pst I site; a Hind III site 31 bp from the EcoRI site; a Hpa I site 39 bp from the Hind III site; a Bam HI site 422 bp from the Hpa I site; a Sal I site 275 bp

from the Bam HI site; and the Pst I site 2254 bp from the Sal I site.

Such constitute further embodiments of the present invention.

pWT 550 may be produced by a process which comprises restricting pWT 501 using Hae III, isolating the trp-promoter-containing fragment, partially restricting this fragment using Taq I, isolating the 100 bp Hae III-- Taq I fragment, repairing the staggered Taq I end of this fragment using DNA polymerase I, ligating the resulting fragment to Hind III linkers, restricting the resulting fragment using Hind III, isolating the trp-promoter-containing fragment, ligating this fragment to Hind III-cut, bacterial alkaline phosphatase-treated pAT 153, transforming E. coli K 12 HB101 using the resulting plasmid and selecting for ampicillin-resistance and tetracycline-resistance.

pWT 560 may be produced by a similar process, except that one would select for ampicillin-resistance and tetracycline-sensitivity.

pWT 551 may be produced by a process which comprises partially restricting pWT 550 using Hind III, isolating the linearised molecule, incubating this molecule with DNA polymerase I, ligating the blunt ends, transforming E. coli K 12 HB101 using the resulting plasmid, selecting for ampicillin-resistance and tetracycline-resistance and identifying the product by restriction enzyme analysis.

pWT 561 may be produced by a similar process using pWT 560 and selecting for ampicillin-resistance and tetracycline-sensitivity.

Further embodiments of the present invention are directed accordingly.

pWT 551, for example, is capable of expressing an inserted gene sequence from its own N-terminus by vitue of placing the sequence immediately downstream of the attenuator peptide SD site via a unique Hind III site.

Multiple promoter derivatives, in particular of pWT 551, have also been constructed.

It has been shown, (see, for example, Backman, K., and Ptashne, M., (1978), Cell, 13, 65-71), that the positioning of two 205 bp lac promoter fragments, (see, for example, Backman, K., et al, (1976), Proc. Natl. Acad. Sci. U.S.A., 73, 4174 - 4178) immediately upstream of a λ CI (repressor) gene, such that both promoters transcribe into the gene, increases the level of repressor produced 2.2-fold over that produced where only a single 205 bp fragment is positioned upstream of the gene. Two reasons may be given as to why this should be. Firstly, titration of the lac repressor caused by the increase in operators would leave more promoters free to initiate transcription and secondly, an increase in the rate of transcription due to RNA polymerase molecules initiating transcription from two promoters rather than one (multiple transcription) with the result that two mRNAs are produced. Such increased transcription would be beneficial in the expression of eukaryotic genes which generally speaking are not expressed as efficiently as genes of prokaryotic origin.

Multiple promoter derivatives of pWT 551 may easily be constructed by repeated cloning at the Hind III site of the 111 bp Hind III trp promoter fragment, followed by removal of the upstream site. Furthermore, if titration of the repressor is the major cause of increased expression by such plasmids, (see, for example, Goeddel, D.V., et al, (1980), Nature, 287, 411 - 416) it should be possible to verify this by constructing multiple promoter plasmids with the expression - cloning site situated at intermediate positions within a series of promoters. Thus, a gene cloned at position 1 in a triple promoter plasmid should be expressed as efficiently as a gene inserted at position 3. On the other hand, if multiple transcription is the reason for increased expression, then a gene inserted at the latter position should result in an increase in expression relative to expression on insertion at position 1.

For purposes of exemplification, the following illustrates the construction of multiple promoter derivatives of pWT 551:

(i) trp promoter clonings

The 111 bp Hind III trp promoter fragment
(recovered from acrylamide on electrophoresing Hind III-
restricted pWT 550) was ligated to Hind III-cut, bacterial
alkaline phosphatase-treated pWT 551 and the ligated
molecules transformed into E. coli K 12 HB101, transformants being selected for on nutrient agar carbenicillin.
A total of 2121 transformants was obtained, from which
12 were chosen for plasmid DNA preparations.

The presence of cloned DNA was demonstrated by
double restriction with Pst I and Bam HI. pWT 551 thus-
restricted produces bands of 2529 and 1245 bp, the
smaller band containing the trp promoter. On cloning
a single 111 bp trp fragment, this band increases in
size to 1356 bp; of the 12 plasmid DNAs restricted, 11
have cloned a single trp fragment. The plasmid digest
found where the smaller band has a size of ∼ 1467 bp,
probably represents a double trp promoter fragment
cloning.

Restriction with Hae III was used to verify the
orientation of the cloned trp promoter(s). The restriction
profile for pWT 551 is as for pWT 550 with the exception
that the 54 bp Hae III fragment has been increased in
size to 58 bp (by polymerase repair at the upstream
Hind III site) and migrates as a dimer with a pAT 153
band of 57 bp. Hae III profiles for a single and double
trp promoter fragment cloning into pWT 551 were prepared.
Interestingly, all 12 plasmids produce an identical profile which would seem to indicate that only one of the orientations is permissible. In addition to the two pWT 551 bands associated with the trp promoter fragment (58 and 248 bp), the digests include a band of 111 bp, furthermore, in the case of the double cloning this band is a dimer. A fragment of 111 bp may only be produced if the trp fragment, which has a single Hae III site situated upstream of the promoter region, inserts at the Hind III site of pWT 551 in the 'R' orientation, two fragments of this size being produced where a double cloning (both 'R' orientated) has occurred.

As a final check, the single and double trp insert plasmids were restricted with Hind III. The restriction profiles confirm the presence within the plasmids of the 111 bp Hind III trp promoter fragment. These two plasmids were designated pWT 551-2P (double trp promoter containing) and pWT 551-3P (triple trp promoter containing).

(ii) Removal of unwanted Hind III sites

The isolation of the triple promoter plasmid, pWT 551-3P, was an unexpected advantageous result as this eliminated the need to clone an additional promoter fragment into pWT 551-2P. Three derivatives of pWT 551-3P, each with a unique Hind III site located at the downstream end of the first, second and third promoter fragment may be constructed by the removal of the two unwanted Hind III sites. Similarly, two derivatives of pWT 551-2P may be constructed by removal of one of the two Hind III sites to leave the cloning - expression site at position 1 or 2.

Removal of a Hind III site was accomplished as in the construction of pWT 511 and pWT 551 by DNA polymerase I repair of partially Hind III-cut pWT 551-2P and pWT 551-3P, followed by religation. More particularly, 2 µg of plasmid DNA was restricted for 60 minutes at 25°C in a reaction volume of 400 µl using 0.6 units of enzyme. The reaction mixture contained 10 mM Tris-HCl, pH 7.5, 10 mM $MgCl_2$, 1 mM DTT, 100 µg/ml gelatin and 50 mM NaCl. The DNA was then extracted with equal volumes of phenol and chloroform and ethanol precipitated. Under these conditions the majority of plasmid molecules were cleaved only once. These linearised molecules were separated from uncut plasmid by means of preparative gel electrophoresis.

The ligated molecules were then transformed into E. coli K 12 HB101 selecting on nutrient agar carbenicillin. Plasmid DNAs prepared from a number of transformants were tested for the loss of one of the Hind III sites by restriction analysis. In this manner, double and triple promoter plasmids having one and two Hind III sites, respectively, were isolated. The above procedure was repeated on the triple promoter isolates to remove a second Hind III site.

Hind III site removal was demonstrated by double restriction with Pst I and Hind III. As expected, the double promoter plasmid with the cloning site situated at position 1 produces a 899 bp fragment as does pWT 551 and a fragment of 2990 bp, this latter fragment being 115 bp (111 bp trp fragment + 4 bp from the polymerase repair) larger than the equivalent pWT 551 fragment (2875 bp). With the position 2 plasmid the 899 bp fragment increases to 1014 bp (899 + 115 bp) and the larger fragment, 2875 bp is as in pWT 551. These digests confirm the presence of a Hind III site at the stated position. The plasmids were named according to the position of the Hind III site, pWT 551-2P1 and pWT 551-2P2, the final number designating the position of the cloning site. Restriction profiles for triple promoter plasmids with the cloning site situated at position 1, 2 and 3 were obtained. The 899 bp fragment of the position 1 plasmid increases by 115 and 330 bp on positioning the cloning site after the second and third promoter fragment, producing bands of 1014 bp and 1129 bp. Similarly, the 2875 bp fragment of the position 3 plasmid increases by the same amounts in position 2 and 1 plasmids to give bands of 2990 bp and 3105 bp. Thus-confirmed as having a Hind III site in the stated position, these plasmids were designated pWT 551-3P1, pWT 551-3P2 and pWT 551-3P3.

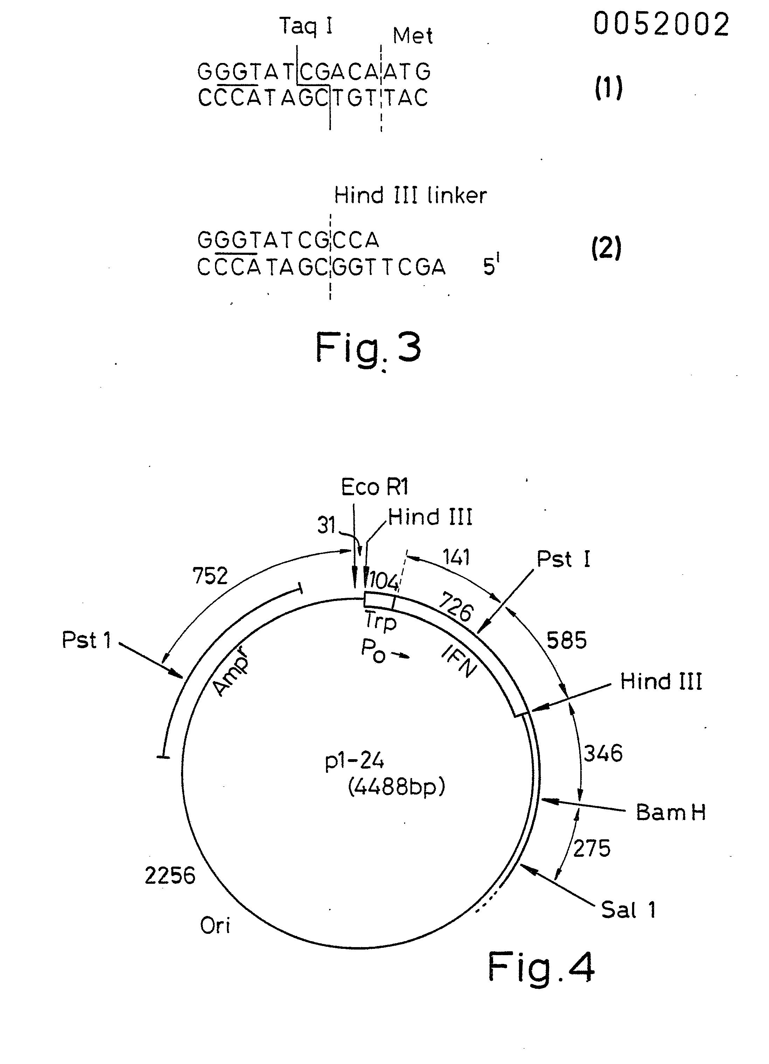

The efficient translation of an inserted gene sequence depends on the correct positioning thereof relative to the ribosome binding site. This may be achieved by removal of unnecessary nucleotides between the initiator codon of the gene sequence prior to its cloning at the expression site. As indicated above, pWT 551 has been constructed and the sequence verified such that S1 nuclease treatment of the cleaved Hind III site (removal of the 5' extension) will leave a blunt DNA end and the same number of nucleotides between the -GGT- sequence in the ribosome binding site and the initiator codon of an inserted gene sequence as with the attenuator peptide (provided that the inserted DNA sequence begins at the initiator, see Figure 3 of the accompanying drawings).

Changes in the distance between the ribosome binding site (GGT) and the initiation codon of the gene to be expressed may be accomplished by using DNA polymerase I in the presence of one or more deoxynucleotide triphosphates, followed by S1 nuclease treatment to produce blunt ends.

Correct translation of gene sequences inserted into the Hind III expression site of pWT 551 is dependent on the presence of an initiator, ATG or GTG, at the start of the coding region. If such an initiator is absent as with many mature polypeptides which do not have an N-terminal methionine, e.g. leukocyte interferon, the sequence cannot be translated. However, translation would be possible if an ATG codon were present within the expression-cloning site immediately preceding the first amino acid of the inserted gene sequence. The translated product would then start with an f-methionine at its N-terminus, followed by the first amino acid of the mature protein. Such a system has been used for the synthesis of mature human growth hormone in E. coli, (see Goeddel, et al, loc cit). A plasmid suitable for this type of expression may be constructed by attaching a synthetic DNA fragment (linker) containing an ATG proceeded by a suitable unique restriction site, e.g. EcoRI or Bam HI, to the DNA polymerase I - repaired Taq I end of the 100 bp Hae III-Taq I fragment used in the construction of pWT 551.

The 100 bp Hae III-Taq I fragment containing the trp-promoter was recovered from 20 µg of pWT 501 and DNA polymerase I - repaired as described above in connection with the construction of pWT 550 and pWT 560. The synthetic 16 bp linker fragment, which was synthesised by conventional methods (see, for example, Hsuing, H.M., et al, (1979), Nucleic Acids Research, 6, 1371-1385), containing a central EcoRI site as follows:

```
 5'                                              3'

      A C A A T G A A T T C A T T G T
      T G T T A C T T A A G T A A C A
```

was ligated to the fragment in a ratio of 50 moles of linker per mole of fragment. (Such a fragment and the production thereof by a process which comprises the chemical synthesis and ligation of appropriate oligonucleotides constitute further embodiments of the present invention.) EcoRI ends were produced by restricting the ligation mixture using EcoRI (EC 3.1.4.32). Unbound linker was removed from the ligated material by chromatography through a 50 x 0.7 cm column of G 150 Sephadex. The excluded peak containing the trp-promoter fragment and attached linkers was pooled and the DNA precipitated. pAT 153 was digested using EcoRI, treated with bacterial alkaline phosphatase and the 100 bp trp fragment.

Ligated molecules were transformed into E. coli K 12 HB101 applying a nutrient agar ampicillin (100 µg/ml) selection. Plasmid DNA's were prepared from a number of transformants. On restriction using EcoRI, these produced a band of approximately 117 bp, the size expected for the cloned fragment, as well as the linearised vector.

The orientation of the trp-promoter was determined by restriction using Taq I. In addition to the internal 42 bp Taq I fragment present within the cloned trp fragment (the Taq I site within the ribosome binding site having been recreated on ligation of the EcoRI linker to the repaired Taq I end), two new fragments will be produced depending on the orientation. Where inserted in the right-ward orientation, the promoter transcribing towards the tetracycline resistance gene, bands of 25 and 418 bp were produced. In the opposite orientation, these were replaced by bands of 82 and 361 bp. The plasmids having these orientations are designated pWT 570 and pWT 580, respectively.

The plasmid designated pWT 570 has a molecular length of 3775 bp; a Pst I site; a first EcoRI site 752 bp from the Pst I site; a Hpa I site 75 bp from the first EcoRI site; a second EcoRI site 42 bp from the Hpa I site; a Hind III site 31 bp from the second EcoRI site; a Bam HI site 346 bp from the Hind III site; a Sal I site 275 bp from the Bam HI site; and the Pst I site 2254 bp from the Sal I site.

The plasmid designated pWT 580 has a molecular length of 3775 bp; a Pst I site; a first EcoRI site 752 bp from the Pst I site; a Hpa I site 42 bp from the first EcoRI site; a second EcoRI site 75 bp from the Hpa I site; a Hind III site 31 bp from the second EcoRI site; a Bam HI site 346 bp from the Hind III site; a Sal I site 275 bp from the Bam HI site; and the Pst I site 2254 bp from the Sal I site.

pWT 570 may be produced by a process which comprises restricting pWT 501 using Hae III, isolating the trp-promoter-containing fragment, partially restricting this fragment using Taq I, isolating the 100 bp Hae III-Taq I fragment, repairing the staggered Taq I end of this fragment using DNA polymerase I, ligating the resulting fragment to EcoRI linkers A C A A T G A A T T C A T T G T, restricting the resulting fragment using EcoRI, isolating the trp-promoter-containing fragment, ligating this fragment to pAT 153, which has been digested using EcoRI and treated with bacterial alkaline phosphatase, transforming E. coli K 12 HB101 using the resulting plasmid, selecting for ampicillin-resistance and/or tetracycline-resistance and identifying the product by restriction enzyme analysis.

pWT 580 may be produced by a similar process.

Such constitute further embodiments of the present invention.

Removal of the upstream EcoRI site was accomplished by DNA polymerase I repair of partially EcoRI-restricted plasmid, followed by recircularisation. pWT 570 was so-treated and the ligation mixture transformed in E. coli K 12 HB101 selecting for Ap$^r$ transformants. Removal of the site was confirmed by restriction using EcoRI and Ava I of plasmid isolates from a number of transformants. Bands of 1426 bp and 2353 bp are produced, which is as predicted for removal of the upstream EcoRI site. The resulting plasmid is designated pWT 571.

As with the plasmids pWT 502 and pWT 560, an analogous procedure may be used to construct the corresponding derivative of pWT 580, pWT 581.

The plasmid designated pWT 571 has a molecular length of 3779 bp; a Pst I site; a Hpa I site 831 bp from the Pst I site; an EcoRI site 42 bp from the Hpa I site; a Hind III site 31 bp from the EcoRI site; a Bam HI site 346 bp from the Hind III site; a Sal I site 275 bp from the Bam HI site; an Ava I site 774 bp from the Sal I site; and the Pst I site 1480 bp from the Ava I site.

The plasmid designated pWT 581 has a molecular length of 3779 bp; a Pst I site; an EcoRI site 752 bp from the Pst I site; a Hpa I site 42 bp from the EcoRI site; a Hind III site 110 bp from the Hpa I site; a Bam HI site 346 bp from the Hind III site; a Sal I site 275 bp from the Bam HI site; an Ava I site 774 bp from the Sal I site; and the Pst I site 1480 bp from the Ava I site.

pWT 571 may be produced by a process which comprises partially restricting pWT 570 using EcoRI, isolating the linearised molecule, incubating this molecule with DNA polymerase I, ligating the blunt ends, transforming E. coli K 12 HB101 using the resulting plasmid, selecting for ampicillin-resistance and/or tetracycline-resistance and identifying the product by restriction enzyme analysis.

pWT 581 may be produced by a similar process using pWT 580.

Such constitute further embodiments of the present invention.

Restriction of pWT 571 using EcoRI, followed by S1 nuclease - DNA polymerase I repair, exposes the ATG codon present within the linker fragment, thereby enabling blunt end ligation of a DNA sequence coding for a protein lacking an N-terminal methionine directly to the initiator codon. The nucleotide composition of the linker is such that the three nucleotides preceding the ATG are as in the trp-leader ribosome binding site, (see Figure 1 of the accompanying drawings), therefore, provided the DNA polymerase I repair of the Taq I end is exact, the complete ribosome bind site should be present as follows:

```
     5'                                        3'

        G G T A T C G A C A A T G

        C C A T A G C T G T T A C T T A A
```

This was demonstrated by (i) restriction using Taq I to show that the Taq I site has been reformed and (ii) sequencing through the EcoRI site (sequencing from Hpa I).

Of course, as mentioned above, the end-to-end joining of fragments to produce promoters in series may be applied to the above plasmids.

Various fragments of the above plasmids containing the trp-promoter may be thought of as the basis of the present invention

Certain particularly interesting fragments are as follows:

```
(1)    C C G A C A T C A T A A C G G T T C -

       G C G G C T G T A G T A T T G C C A A G -
```

```
          TGGCAAATATTCTGAAATGAG-
          ACCGTTTATAAGACTTTACTC-

          CTGTTGACAATTAATCATCGAAC-
          GACAACTGTTAATTAGTAGCTTG-

          TAGTTAACTAGTACGCAAGTTC-
          ATCAATTGATCATGCGTTCAAG-

          ACGTAAAAAGGGTATCGACAATG-
          TGCATTTTTCCCATAGCTGTTAC-

          AAAGCAATTTTCGTACTGAAAGGT-
          TTTCGTTAAAAGCATGACTTTCCA-

          TGGTGGCG
          ACCACC

     (2)        CCGACATCATAACGGTTC-
                 GGCTGTAGTATTGCCAAG-

          TGGCAAATATTCTGAAATGAG-
          ACCGTTTATAAGACTTTACTC-

          CTGTTGACAATTAATCATCGAAC-
          GACAACTGTTAATTAGTAGCTTG-

          TAGTTAACTAGTACGCAAGTTC-
          ATCAATTGATCATGCGTTCAAG-

          ACGTAAAAAGGGTATCGACAATG-
          TGCATTTTTCCCATAGCTGTTAC-
```

```
A A A G C A A T T T T C G T A C T G A A A G G T -
T T T C G T T A A A A G C A T G A C T T T C C A -

T G G T G G
A C C A C C

(3)          C C G A C A T C A T A A C G G T T C -
             G G C T G T A G T A T T G C C A A G -

T G G C A A A T A T T C T G A A A T G A G -
A C C G T T T A T A A G A C T T T A C T C -

C T G T T G A C A A T T A A T C A T C G A A C -
G A C A A C T G T T A A T T A G T A G C T T G -

T A G T T A A C T A G T A C G C A A G T T C -
A T C A A T T G A T C A T G C G T T C A A G -

A C G T A A A A A G G G T A T
T G C A T T T T T C C C A T A G C
```

Such fragments may be produced by a process which comprises the chemical synthesis and ligation of appropriate oligonucleotides.

Alternatively, such fragments may be produced by a process which comprises restricting a suitable plasmid using one or more appropriate enzymes.

More particularly, fragment (1) may be obtained by restricting pWT 221 using Hha I; fragment (2) may be obtained by restricting pWT 501 using Hae III; and fragment (3) may be obtained by restricting pWT 501 using Hae III and partially restricting using Taq I.

Such constitute further embodiments of the present invention.

The present invention further provides such a plasmid having inserted therein at the insertion site a DNA fragment, in particular a eukaryotic DNA fragment, and the production thereof.

The present invention further provides a cell, in particular an E. coli K 12 HB101 cell, which has been transformed by having inserted therein such a plasmid and the production thereof.

The present invention further provides a process for the expression of a protein which comprises culturing such a cell.

For example, a Hind III fragment of 1728 bp containing the FPV HA gene, (see, for example, Emtage, J.S., et al, (1980), Nature, 283, 171-174), has been cloned into the Hind III site of pWT 511, such that it is under trp-promoter control, and transformed into E. coli K 12 HB101 where expression results.

The following exemplifies this embodiment of the present invention.

In addition to the 558 amino acid HA-coding sequence within the 1728 bp Hind III FPV-HA fragment, there is also present immediately preceeding the ATG of the HA-prepeptide a region which is partly complementary

to the 3' end of the E. coli 16S ribosomal RNA and enables ribosome binding and translation directly from the ATG, (see Emtage, et al, loc cit).

The presence of this prokaryotic-like ribosome binding site therefore eliminates the need to express the HA-gene as part of a fusion peptide in a correctly phased expression plasmid, or indeed, to process the DNA prior to cloning such that the ATG is placed adjacent to an E. coli SD site (as would be necessary for expression in pWT 551). However, the ribosome binding site is only recognised with an efficiency of ~20% compared to the E. coli site. Thus, insertion of the gene at the Hind III site of pWT 121, which is in-phase for correct translation of the HA as a fusion with the trp E amino acids, results in the production of 20.3 ng of HA (per 50 μl of bacterial lysate) when partially derepressed for trp controlled expression compared to 5.0 ng of HA on expression in pWT 111 which is out-of-phase, (see Emtage, et al, loc cit).

In theory, an 8 to 10-fold increase in product might be expected in an attenuator deleted trp plasmid, (see Miozzari and Yanofsky, loc cit), but, in practice, in view of the probable membrane binding nature of the HA protein via the hydrophobic C terminus, (see, for example, Porter, A.G., et al, (1979), Nature, 282, 471-477), this may not be attained. It was therefore decided to clone the gene into pWT 511 for out-of-phase expression (pWT 511 has the same phasing as pWT 111) from the less efficient prokaryotic-like ribosome binding site with a view to changing the phase (if practicable) across the fusion junction to increase expression even further.

FPV Hind III DNA was ligated to Hind III-cut. pWT 511, transformed into E. coli K 12 HB101 and recombinant colonies selected applying a nutrient agar, carbenicillin and tryptophan (100 µg/ml) selection. A total of 29 transformants were obtained and were screened for plasmid size on whole cell gels. Single colonies were screened for plasmid DNA by known methods (see Twigg and Sherratt, loc cit).

A representation sample of cells from an agar streak of a single colony (enough cells to fill a 1 µl innoculating loop) was re-suspended in 250 µl of agarose gel electrophoresis buffer (AGEB) containing 1% w/v SDS, 2% w/v Ficoll 70 (Sigma; copolymer of sucrose and epichlorohydrin) and 0.01% w/v bromophenol blue (Sigma). The mixture was incubated for 30 minutes at 65°C. 5 µl of ribonuclease IA was then added and the lysate vortexed for 30 seconds. A 50 µl fraction of this lysate was found sufficient for plasmid identification on a 1% w/v gel.

26 contained full size R orientated plasmids. These recombinants were further screened for FPV gene orientation by testing for resistance to tetracycline on

tetracycline (10 µg/ml) and carbenicillin-supplemented M9-casamino acids minimal agar $\pm$ tryptophan (100 µg/ml). An even distribution of probable L and R insertions was found. 11 recombinants were $Tc^S \pm$ tryptophan and were probably L insertions, while the remaining 12 recombinants were $Tc^r$ plus tryptophan, but failed to grow in its absence (the same was observed minus Tc), probable R insertions. Failure of the R orientated FPV plasmid to grow in the absence of tryptophan would suggest over-production of HA on partial derepression, a situation not encountered with pWT 221/FPV(R) in $\underline{E}$. $\underline{coli}$ K 12 HB101. Plasmid DNAs were prepared from $4Tc^r$ and $4Tc^S$ recombinant colonies for restriction analysis.

On restriction with Hind III, all 8 plasmids produced the 1728 bp FPV-HA fragment. Orientation was confirmed by restriction with Pst I. All 4 $Tc^r$ plasmids produced bands of 3425 and 2108 bp confirming the R orientation of the FPV insert. This plasmid was designated pWT 511/FPV(R). The $Tc^S$ plasmids produced bands 4053 and 1480 bp, thus confirming correct L insertion.

A determining factor in the choice of FPV-HA production as a means of assessing the potential of the attenuator deleted $\underline{trp}$ expression plasmid pWT 511 was that a sensitive assay for the HA ($\underline{E}$. $\underline{coli}$) antigen was available. This is based on a solid phase antibody-antigen binding radioimmunoassay which uses iodinated antibody raised against the virus.

More particularly, the HA-antigen assay used was a modification of a known antibody sandwich technique, (see, for example, Broome, S., and Gilbert, W., (1978), Proc. Natl. Acad. Sci. U.S.A., _75_, 2746-2749) whereby HA-antigen in bacterial lysates was detected by the binding thereof to FPV-HA specific $^{125}$I-IgG.

Plasmid-containing cells to be screened for HA-antigen were grown in 5 ml of M9-casamino acids minimal medium supplemented with 100 µg/ml of carbenicillin (Beecham) for up to 8 hours (cells were normally grown to an O.D.$^{600}$ of 0.3; however, if this was not achieved the incubation was continued for up to 8 hours). Additional supplements (IAA or tryptophan) were also present as indicated. The cells were pelleted by centrifugation and lysed in a final volume of 1 ml using a lysozyme, Triton (Registered Trade Mark) X-100 technique, (Triton X-100 is a non-ionic detergent, isooctylphenoxypolyethoxyethanol).

For example, cells were pelleted and re-suspended in 300 µl of ice cold 25% w/v sucrose in 0.05 M Tris-HCl, pH 8.0. All further reactions were performed on ice. 100 µl of 10 mg/ml lysozyme was added and the tube swirled for 5 minutes, followed by addition of 100 µl of 0.4 M EDTA (ethylene diamine tetra-acetic acid disodium salt), pH 8.5. After swirling for 5 minutes, lysis was brought about by the addition of 500 µl of "lytic mix" (2% v/v Triton X-100, 0.05 M Tris-HCl, pH 8)

and the tube swirled for a further 20 minutes. The lysates were directly assayed for HA-antigen activity without further treatment.

Assays were performed in polystyrene culture tubes (Nunc no. 1410) which had been treated with purified rabbit FPV-HA specific IgG. The IgG component was isolated by ammonium sulphate precipitation and diethylaminoethyl (DEAE)-cellulose DE 52 chromatography, (see, for example Livingston, D.M., (1974), Methods Enzymol., 34, 723-731), and labelled with $^{125}$I, (see, for example, Hunter, W.M., and Greenwood, F.C., (1964), Brit. Med. J., 5386, 804-807). Tubes were coated with 50 µl of 0.2 M NaHCO$_3$, pH 9, containing 60 µg/ml purified, unlabelled IgG for 10 minutes at room temperature, washed with 3 x 1 ml aliquots of wash buffer (0.2 M NaCl, 50 mM KH$_2$PO$_4$, pH 7.4, 0.1% w/v BSA (bovine serum allumin), 0.1% v/v NP-40 (Nonidet P40, BDH) and 0.5% normal goat serum) and then incubated at room temperature either with known amounts (1 - 20 ng) of Sarkosyl-disrupted FPV or with the bacterial lysates. After 2 hours, the tubes were washed with 4 x 1 ml aliquots of wash buffer and finally incubated at 4°C for 16 hours with 50 µl wash buffer containing 200,000 c.p.m. $^{125}$Ianti-HA. Tubes were again washed and counted in a Packard ɣ-counter. Antigen present was quantified from standard curves relating the radioactivity bound to the amount of FPV present.

E. coli K 12 HB101 cells separately harbouring pWT 511/FPV(R) and pWT 121/FPV(R) were initially assayed for HA antigen production in M9-casamino acids carbenicillin minimal medium with added tryptophan. In the case of pWT 511/FPV(R), cells were grown at two concentrations of tryptophan, 10 and 100 µg/ml. pWT 121/FPV(R) cells were grown with only 100 µg/ml tryptophan. Growth was monitored at hourly intervals to an $OD^{600}$ of 0.3 (approximately 3 hours), the cells pelleted, lysed with lysozyme and Triton X-100 and 50 µl of the lysate assayed for HA content. No difference was noted between the growth rates at 10 and 100 µg/ml tryptophan for pWT 511/FPV(R) cells. The results of the assays indicate little difference between the amounts of HA produced at the two tryptophan concentrations used with pWT 511/FPV(R). Both values are considerably higher than that for repressed pWT 121/FPV(R); at $Trp^{10}$ the attenuator minus plasmid produces 6.4-fold more HA than the former and 5.8-fold more at $Trp^{100}$. It has been found that the out-of-phase FPV recombinant pWT 111/FPV(R) produced, as anticiptated, even less HA at $Trp^{100}$, 0.157 ng HA/50 µl. If this figure is used as the basal level, then pWT 511/FPV(R) produces 10.2 and 9.3-fold more HA at $Trp^{10}$ and $Trp^{100}$, respectively. From these results, it is clear that pWT 511/FPV(R) is expressing the HA gene to a higher degree than either of the two attenuator intact FPV plasmids. Furthermore, the HA values attained appear not to be in part due to increased copy numbers, as both pWT 511/FPV(R) and (L) plasmids have a copy number similar to that of a pBR 322 (see, for example, Bolivar, F., et al, (1977), Gene, 2, 95-113) based replicon rather than pAT 153-based.

In conclusion, the expression of the FPV-HA gene in pWT 511 has shown this plasmid to be a more efficient expressor than plasmids which include an attenuator.

As suggested above, the use of multiple promoters offers certain advantages. For purposes of exemplification, the following illustrates the use of a triple promoter plasmid:

The 830 bp Hind III fragment from the plasmid p1-24, (see Figure 4 of the accompanying drawings and Figure 11 of published U.K. Patent Application No. 2,068,970), which contains the trp p/o and the FIF gene, was cloned into the Hind III site of pWT 551-2P2, selecting for transformants on nutrient agar supplemented with ampicillin (100 µg/ml). DNAs were prepared and an R orientated insert identified by restriction using Pst I (fragment sizes 1028 and 3462 bp).

Such a construction places the interferon gene under the control of three trandemnly-arranged trp promoters.

E. coli K 12 HB101 cells separately harbouring p1-24 and the triple promoter derivative, pWT 551-2P2/ trp FIF, were induced to produce interferon.  150 ml cultures of each clone were grown in L-broth (luria broth: 1% w/v bacto tryptone, 0.5% w/v bacto yeast extract, 0.5% w/v NaCl, 0.2% w/v glucose, 0.004% w/v thymine, pH 7 (N NaOH)) containing ampicillin to an O.D.$_{600\ nm}$ of about 0.3.  The bacteria were pelleted by centrifugation, washed and then re-suspended in inducing medium (42 mM $Na_2HPO_4$, 22 mM $KH_2PO_4$, 8.6 mM NaCl, 18.7 mM $NH_4Cl$, 0.1 mM $CaCl_2$, 1.0 mM $MgSO_4$, 1 µg/ml vitamin B1, 0.2% w/v glucose, 0.5% w/v casamino acids lacking tryptophan (Difco) and 100 µg/ml ampicillin).  Following incubation at 37°C for 4 hours (at which time the O.D.$_{600\ nm}$ was about 1.0) the cells were centrifuged and extracted.  The extraction technique involved sonicating the bacteria in 10 mM Tris-HCl, pH 8.0, followed by centrifugation for 30 minutes at 15,000 x g and collecting the supernatant for assay.  Assay for interferon activity was carried out by monitoring the protection conferred on Vero (African green monkey) cells against the cpe (cytopathic effect) of EMC (Encephalomyocaditis) virus in an in vitro micro-plate assay system, (see, for example, Dahl, H., and Degre, M., (1972), Acta, Path. Microbiol. Scan., 1380, 863).

Yield              pl-24   5.8  x $10^5$ units/l

pWT 551-2P2/trp FIF  1.95 x $10^6$ units/l

(1 unit being defined in terms of that amount which results in a 50% reduction of viral replication in tissue culture).

-64-                                        0052002

About three times the yield, as determined
by anti-viral activity, results from the triple
promoter plasmid.

As will be appreciated from the foregoing,
inter alia, the present invention has a practical
utility in that it enables useful proteins, which
would otherwise be difficult or impossible to synthesise,
to be produced in improved yields.

CLAIMS:

1.        A plasmid having an insertion site for a DNA fragment adjacent to a bacterial promoter and downstream from a prokaryotic ribosome binding site and optionally an initiator codon such that an inserted DNA fragment is under bacterial promoter control, characterised in that the attenuator of the promoter has been removed.

2.        A plasmid as claimed in claim 1 characterised in that the promoter is a trp-promoter.

3.        A plasmid as claimed in claim 1 or claim 2 characterised in that it has multiple promoters.

4.        A plasmid as claimed in claim 1 or claim 2 characterised in that it has a molecular length of 3805 bp; a Pst I site; an EcoRI site 752 bp from the Pst I site; a first Hind III site 31 bp from the EcoRI site; a Hpa I site 72 bp from the first Hind III site; a second Hind III site 75 bp from the Hpa I site; a Bam HI site 346 bp from the second Hind III site; a Sal I site 275 bp from the Bam HI site; and the Pst I site 2254 bp from the Sal I site; the plasmid being designated pWT 501; or characterised in that it has a molecular length of 3805 bp; a Pst I site; an EcoRI site 752 bp from the Pst I site; a first Hind III site 31 bp from the EcoRI site; a Hpa I site 75 bp from the first Hind III site; a second
Hind III site 72 bp from the Hpa I site; a Bam HI site
346 bp from the second Hind III site; a Sal I site 275 bp
from the Bam HI site; and the Pst I site 2254 bp from the
Sal I site; the plasmid being designated pWT 502; or
characterised in that it has a molecular length of 3809 bp;
a Pst I site; an EcoRI site 752 bp from the Pst I site;
a Hpa I site 107 bp from the EcoRI site; a Hind III site
75 bp from the Hpa I site; a Bam HI site 346 bp from the
Hind III site; a Sal I site 275 bp from the Bam HI site;
and the Pst I site 2254 bp from the Sal I site; the
plasmid being designated pWT 511; or characterised in that
it has a molecular length of 3809 bp; a Pst I site; an
EcoRI site 752 bp from the Pst I site; a Hind III site
31 bp from the EcoRI site; a Hpa I site 75 bp from the
Hind III site; a Bam HI site 422 bp from the Hpa I site;
a Sal I site 275 bp from the Bam HI site; and the Pst I
site 2254 bp from the Sal I site; the plasmid being
designated pWT 512; or characterised in that it has a
molecular length of less than 3809 bp; a Pst I site; an
EcoRI site 752 bp from the Pst I site; a Hpa I site 107 bp
from the EcoRI site; a Hind III site less than 75 bp from
the Hpa I site; a Bam HI site 346 bp or less from the Hind
III site; a Sal I site 275 bp from the Bam HI site; and
the Pst I site 2254 bp from the Sal I site; the plasmid
being designated pWT 521; or characterised in that it
has a molecular length of less than 3809 bp; a Pst I site;

an EcoRI site 752 bp from the Pst I site; a Hind III
site 31 bp or less from the EcoRI site; a Hpa I site
less than 75 bp from the Hind III site; a Bam HI site
422 bp from the Hpa I site; a Sal I site 275 bp from
the Bam HI site; and the Pst I site 2254 bp from the
Sal I site; the plasmid being designated pWT 522; or
characterised in that it has a molecular length of
less than 3809 bp; a Pst I site; an EcoRI site 752 bp
from the Pst I site; a Hpa I site 107 bp from the
EcoRI site; a Hind III site less than 75 bp from the
Hpa I site; a Bam HI site 346 bp or less from the Hind
III site; a Sal I site 275 bp from the Bam HI site; and
the Pst I site 2254 bp from the Sal I site; the plasmid
being designated pWT 531; or characterised in that it
has a molecular length of less than 3809 bp; a Pst I
site; an EcoRI site 752 bp from the Pst I site; a Hind
III site 31 bp or less from the EcoRI site; a Hpa I site
less than 75 bp from the Hind III site; a Bam HI site
422 bp from the Hpa I site; a Sal I site 275 bp from the
Bam HI site; and the Pst I site 2254 bp from the Sal  I
site; the plasmid being designated pWT 532; or characterised in that it has a molecular length of 3769 bp; a Pst
I site; an EcoRI site 752 bp from the Pst I site; a first
Hind III site 31 bp from the EcoRI site; a Hpa I site 72 bp
from the first Hind III site; a second Hind III site
39 bp from the Hpa I site; a Bam HI site 346 bp from the
second Hind III site; a Sal I site 275 bp from the Bam HI site; and the Pst I site 2254 bp from the Sal I site;
the plasmid being designated pWT 550; or characterised
in that it has a molecular length of 3769 bp; a Pst I
site; an EcoRI site 752 bp from the Pst I site; a first
Hind III site 31 bp from the EcoRI site; a Hpa I site
39 bp from the first Hind III site; a second Hind III
site 72 bp from the Hpa I site; a Bam HI site 346 bp from
the second Hind III site; a Sal I site 275 bp from the
Bam HI site; and the Pst I site 2254 bp from the Sal I
site; the plasmid being designated pWT 560; or characterised in that it has a molecular length of 3773 bp; a Pst I
site; an EcoRI site 752 bp from the Pst I site; a Hpa I
site 107 bp from the EcoRI site; a Hind III site 39 bp
from the Hpa I site; a Bam HI site 346 bp from the Hind
III site; a Sal I site 275 bp from the Bam HI site; and
the Pst I site 2254 bp from the Sal I site; the plasmid
being designated pWT 551; or characterised in that it
has  a molecular length of 3773 bp; a Pst I site; an
EcoRI site 752 bp from the Pst I site; a Hind III site
31 bp from the EcoRI site; a Hpa I site 39 bp from the
Hind III site; a Bam HI site 422 bp from the Hpa I site;
a Sal I site 275 bp from the Bam HI site; and the Pst
I site 2254 bp from the Sal I site; the plasmid being
designated pWT 561; or characterised in that it has a
molecular length of 3775 bp; a Pst I site; a first
EcoRI site 752 bp from the Pst I site; a Hpa I site 75 bp
from the first EcoRI site; a second EcoRI site 42 bp from the Hpa I site; a Hind III site 31 bp from the second EcoRI site; a Bam HI site 346 bp from the Hind III site; a Sal I site 275 bp from the Bam HI site; and the Pst I site 2254 bp from the Sal I site; the plasmid being designated pWT 570; or characterised in that it has a molecular length of 3775 bp; a Pst I site; a first EcoRI site 752 bp from the Pst I site; a Hpa I site 42 bp from the first EcoRI site; a second EcoRI site 75 bp from the Hpa I site; a Hind III site 31 bp from the second EcoRI site; a Bam HI site 346 bp from the Hind III site; a Sal I site 275 bp from the Bam HI site; and the Pst I site 2254 bp from the Sal I site; the plasmid being designated pWT 580; or characterised in that it has a molecular length of 3779 bp; a Pst I site; a Hpa I site 831 bp from the Pst I site; an EcoRI site 42 bp from the Hpa I site; a Hind III site 31 bp from the EcoRI site; a Bam HI site 346 bp from the Hind III site; a Sal I site 275 bp from the Bam HI site; a Ava I site 774 bp from the Sal I site; and the Pst I site 1480 bp from the Ava I site; the plasmid being designated pWT 571; or characterised in that it has a molecular length of 3779 bp; a Pst I site; an EcoRI site 752 bp from the Pst I site; a Hpa I site 42 bp from the EcoRI site; a Hind III site 110 bp from the Hpa I site; a Bam HI site 346 bp from the Hind III site; a Sal I site 275 bp from the Bam HI site; an Ava I site 774 bp from the Sal I site; and the Pst I site 1480 bp from the Ava I site; the plasmid being designated pWT 581.

5.      A process for the production of a plasmid as claimed in any of claims 1 to 4 characterised in that it comprises cloning a bacterial promoter, the attenuator of which has been removed, into an isolated plasmid, determining the position of an intended insertion site adjacent to and downstream from the promoter and providing the insertion site if it is not present.

6.      A process as claimed in claim 5 characterised in that for the production of pWT 501 it comprises restricting pWT 221 using Hha I, isolating the trp-promoter-containing fragment, incubating this fragment with DNA polymerase I, ligating the resulting blunt-ended fragment to Hind III linkers, restricting the ligated material using Hind III, isolating the trp-promoter-containing fragment, ligating this fragment to Hind III-cut, bacterial alkaline phosphatase-treated pAT 153, transforming E. coli K 12 HB101 using the resulting plasmid and selecting for ampicillin-resistance and tetracycline-resistance; or characterised in that for the production of pWT 502 it comprises restricting pWT 221 using Hha I, isolating the trp-promoter-containing fragment, incubating this fragment with DNA polymerase I, ligating the resulting blunt-ended fragment to Hind III linkers, restricting the ligated material using Hind III, isolating the trp-promoter-containing fragment, ligating
this fragment to Hind III-cut, bacterial alkaline
phosphatase-treated pAT 153, transforming E. coli K 12
HB101 using the resulting plasmid and selecting for
ampicillin-resistance and tetracycline-sensitivity; or
characterised in that for the production of pWT 511 it
comprises partially restricting pWT 501 using Hind III,
isolating the linearised molecule, incubating this
molecule with DNA polymerase I, ligating the blunt ends,
transforming E. coli K 12 HB101 using the resulting
plasmid, selecting for ampicillin-resistance and
tetracycline-resistance and identifying the product
by restriction enzyme analysis; or characterised in
that for the  production of pWT 512 it comprises partially
restricting pWT 502 using Hind III, isolating the
linearised molecule, incubating this molecule with DNA
polymerase I, ligating the blunt-ends, transforming
E. coli K 12 HB101 using the resulting plasmid, selecting
for ampicillin-resistance and tetracycline-sensitivity
and identifying the product by restriction enzyme
analysis;  or characterised in that for the production
of pWT 521 or pWT 531 it comprises restricting pWT 511
using Hind III, digesting the exposed Hind III end(s)
of the resulting linear molecule using exonuclease III
and S1 nuclease, repairing the resulting staggered
end(s) using DNA polymerase I, ligating to Hind III
linkers,  transforming E. coli K 12 HB101 using the resulting plasmid, selecting for ampicillin-resistance
and tetracycline-resistance and identifying the phase-
changed product; or characterised in that for the
production of pWT 522 or pWT 532 it comprises restricting
pWT 512 using Hind III, digesting the exposed Hind III
end(s) of the resulting linear molecule using exonuclease
III and S1 nuclease, repairing the resulting staggered
end(s) using DNA polymerase I, ligating to Hind III
linkers, transforming E. coli K 12 HB101 using the
resulting plasmid, selecting for ampicillin-resistance
and tetracycline-sensitivity and identifying the phase-
changed product; or characterised in that for the
production of pWT 550 it comprises restricting pWT 501
using Hae III, isolating the trp-promoter-containing
fragment, partially restricting this fragment using Taq I,
isolating the 100 bp Hae III-Taq I fragment, repairing the
staggered Taq I end of this fragment using DNA polymerase
I, ligating the resulting fragment to Hind III linkers,
restricting the resulting fragment using Hind III,
isolating the trp-promoter-containing fragment, ligating
this fragment to Hind III-cut, bacterial alkaline
phosphatase-treated pAT 153, transforming E. coli K 12
HB101 using the resulting plasmid and selecting for
ampicillin-resistance and tetracycline-resistance;
or characterised in that for the production of pWT 560
it comprises restricting pWT 501 using Hae III, isolating
the trp-promoter-containing fragment, partially restricting this fragment using Taq I, isolating the
100 bp Hae III-Taq I fragment, repairing the staggered
Taq I end of this fragment using DNA polymerase I,
ligating the resulting fragment to Hind III linkers,
restricting the resulting fragment using Hind III,
isolating the trp-promoter-containing fragment, ligating
this fragment to Hind III-cut, bacterial alkaline
phosphatase-treated pAT 153, transforming E. coli K 12
HB101 using the resulting plasmid and selecting for
ampicillin-resistance and tetracycline-sensitivity;
or characterised in that for the production of pWT 551
it comprises partially restricting pWT 550 using Hind
III, isolating the linearised molecule, incubating this
molecule with DNA polymerase I, ligating the blunt ends,
transforming E. coli K 12 HB101 using the resulting
plasmid, selecting for ampicillin-resistance and
tetracycline-resistance and identifying the product by
restriction enzyme analysis; or characterised in that
for the production of pWT 561 it comprises partially
restricting pWT 560 using Hind III, isolating the
linearised molecule, incubating this molecule with
DNA polymerase I, ligating the blunt ends, transforming
E. coli K 12 HB101 using the resulting plasmid, selecting
for ampicillin-resistance and tetracycline-sensitivity
and identifying the product by restriction enzyme
analysis; or characterised in that for the production
of pWT 570 it comprises restricting pWT 501 using Hae III, isolating the trp-promoter-containing fragment, partially restricting this fragment using Taq I, isolating the 100 bp Hae III-Taq I fragment, repairing the staggered Taq I end of this fragment using DNA polymerase I, ligating the resulting fragment to EcoRI linkers A C A A T G A A T T C A T T G T, restricting the resulting fragment using EcoRI, isolating the trp-promoter-containing fragment, ligating this fragment to pAT 153, which has been digested using EcoRI and treated with bacterial alkaline phosphatase, transforming E. coli K 12 HB101 using the resulting plasmid, selecting for ampicillin-resistance and/or tetracycline-resistance, and identifying the product by restriction enzyme analysis; or characterised in that for the production of pWT 580 it comprises restricting pWT 501 using Hae III, isolating the trp-promoter-containing fragment, partially restricting this fragment using Taq I, isolating the 100 bp Hae III-Taq I fragment, repairing the staggered Taq I end of this fragment using DNA polymerase I, ligating the resulting fragment to EcoRI linkers A C A A T G A A T T C A T T G T, restricting the resulting fragment using EcoRI, isolating the trp-promoter-containing fragment, ligating this fragment to pAT 153, which has been digested using EcoRI and treated with bacterial alkaline phosphatase, transforming E. coli K 12 HB101 using the resulting plasmid, selecting for ampicilline-resistance and/or tetracycline-resistance and identifying the product by restriction enzyme analysis; or characterised in that for the production of pWT 571 it comprises partially restricting pWT 570 using EcoRI, isolating the linearised molecule, incubating this molecule with DNA polymerase I ligating the blunt ends, transforming E. coli K 12 HB101 using the resulting plasmid, selecting for ampicillin-resistance and/or tetracycline-resistance and identifying the product by restriction enzyme analysis; or characterised in that for the production of pWT 581 it comprises partially restricting pWT 580 using EcoRI, isolating the linearised molecule, incubating this molecule with DNA polymerase I, ligating the blunt ends, transforming E. coli K 12 HB101 using the resulting plasmid, selecting for ampicillin-resistance and/or tetracycline-resistance and identifying the product by restriction enzyme analysis.

7.      A plasmid as claimed in any of claims 1 to 4 characterised in that it has inserted therein at the insertion site a DNA fragment.

8.      A process for the production of a plasmid as claimed in claim 7 characterised in that it comprises inserting a DNA fragment at the insertion site of a plasmid as claimed in any of claims 1 to 4.

9.      A cell characterised in that it has been transformed by having inserted therein a plasmid as claimed in claim 7.

10.     A cell as claimed in claim 9 characterised
in that it is an E. coli K 12 HB101 cell.

11.     A process for the transformation of a cell
characterised in that it comprises inserting therein
a plasmid as claimed in claim 7.

12.     A process for the expression of a protein
characterised in that it comprises culturing a cell as
claimed in claim 9 or claim 10.

13.     A fragment of a plasmid as claimed in any of
claims 1 to 4 characterised in that it comprises the
bacterial promoter.

14.     A fragment as claimed in claim 13 characterised
in that it comprises the following sequence:
    C C G A C A T C A T A A C G G T T C -
G C G G C T G T A G T A T T G C C A A G -

T G G C A A A T A T T C T G A A A T G A G -
A C C G T T T A T A A G A C T T T A C T C -

C T G T T G A C A A T T A A T C A T C G A A C -
G A C A A C T G T T A A T T A G T A G C T T G -

T A G T T A A C T A G T A C G C A A G T T C -
A T C A A T T G A T C A T G C G T T C A A G -

A C G T A A A A A G G G T A T C G A C A A T G -

T G C A T T T T T C C C A T A G C T G T T A C -

A A A G C A A T T T T C G T A C T G A A A G G T -

T T T C G T T A A A A G C A T G A C T T T C C A -

T G G T G G C G

A C C A C C

15.        A fragment as claimed in claim 13 characterised
in that it comprises the following sequence:

C C G A C A T C A T A A C G G T T C -

G G C T G T A G T A T T G C C A A G -

T G G C A A A T A T T C T G A A A T G A G -

A C C G T T T A T A A G A C T T T A C T C -

C T G T T G A C A A T T A A T C A T C G A A C -

G A C A A C T G T T A A T T A G T A G C T T G -

T A G T T A A C T A G T A C G C A A G T T C -

A T C A A T T G A T C A T G C G T T C A A G -

A C G T A A A A A G G G T A T C G A C A A T G -

T G C A T T T T T C C C A T A G C T G T T A C -

A A A G C A A T T T T C G T A C T G A A A G G T -

T T T C G T T A A A A G C A T G A C T T T C C A -

T G G T G G

A C C A C C

16.        A fragment as claimed in claim 13 characterised in that it comprises the following sequence:

C C G A C A T C A T A A C G G T T C -
G G C T G T A G T A T T G C C A A G -

T G G C A A A T A T T C T G A A A T G A G -
A C C G T T T A T A A G A C T T T A C T C -

C T G T T G A C A A T T A A T C A T C G A A C -
G A C A A C T G T T A A T T A G T A G C T T G -

T A G T T A A C T A G T A C G C A A G T T C -
A T C A A T T G A T C A T G C G T T C A A G -

A C G T A A A A A G G G T A T
T G C A T T T T T C C C A T A G C

17.        A fragment characterised in that it comprises the following sequence:

A C A A T G A A T T C A T T G T
T G T T A C T T A A G T A A C A

18.        A process for the production of a fragment as claimed in any of claims 13 to 16 characterised in that it comprises restricting a suitable plasmid using one or more appropriate enzymes.

19.        A process for the production of a fragment as claimed in any of claims 13 to 17 characterised in that it comprises the chemical synthesis and ligation of appropriate oligonucleotides.

20.      A process as claimed in claim 18 for the production of a fragment as claimed in claim 14 characterised in that it comprises restricting pWT 221 using Hha I.

21.      A process as claimed in  claim 18 for the production of a fragment as claimed in claim 15 characterised in that it comprises restricting pWT 501 using Hae III.

22.      A process as claimed in claim 18 for the production of a fragment as claimed in claim 16 characterised in that it comprises restricting pWT 501 using Hae III and partially restricting using Taq I.

Hha I

GCG|CCGA
C|GCGGCT

Hind III     Hae III                                                                          Hinc II

A|AGCTTGG|CCGACATCATAACGGTTCTGGCAAATATTCTGAAATGAGCTGTT|GACAATTAAT-
TTCGA|ACC|GGCTGTAGTATTGCCAAGACCGTTTATAAGACTTACTCGACAA|CTGTTAATTA-

              Hinc II                                          Transcription →                Taq I        1    2    3
Taq I         Hpa I                                          * Transcription          int                  Met  Lys  Ala

-CAT|CGAACTAGTT|AACTAGTACGCAAGTTCACGTAAAAAGGGTAT|CGACAATG, AAA, GCA, -
-GTAGC|TTGATCAA|TTGATCATGCGTTCAAGTGCA|TTTTTCCCATAGC|TGTTAC TTT CGT -

                                                                      ribosome protection

4     5     6     7     8     9     10    11    12
Ile   Phe   Val   Leu   Lys   Gly   Trp   Trp   Pro   Hind III

-ATT, TTC, GTA, CTG, AAA, GGT, TGG TGG|, CCA, |AGCTT
-TAA  AAG  CAT  GAC  TTT  CCA  ACC ACC|  GGT  TCGA|A

                                        Hae III
                                        Bal I

                                        Trp
                                        TGG, CG|C, ACT
                                        ACC |GCG TGA

                                        Hha I

# Fig.1

Hae III

GG|CCGACATCATAACGGTTCTGGCAAATATTCTGAAATGAGCTGTTGACAATTAAT-
CC|GGCTGTAGTATTGCCAAGACCGTTTATAAGACTTTACTCGACAACTGTTAATTA-

──────────────────────────── 58bp ────────────────────────────

Taq I                                    *          ───────────▶          Taq I        1    2

-CAT|CGAACTAGTTAACTAGTACGCAAGTTCACGTAAAAAGGGTAT|CGACAATG, AAA,-
-GTAG|C TTGATCAATTGATCATGCGTTCAAGTGCATTTTTCCCATAGC|TGTTAC TTT -

──── ◀─────────────────── 42bp ───────────────────▶ ────

3     4     5     6     7     8     9     10    11   Hae III

-GCA, ATT, TTC, GTA, CTG, AAA, GGT, TGG, TGG|CC
-CGT  TAA  AAG  CAT  GAC  TTT  CCA  ACC  ACC|GG

◀───────────── 37bp ─────────────▶

Fig.2

0052002

Taq I     Met

GGGTAT|CGACA|ATG          (1)
CCCATAGC|TGT|TAC

Hind III linker

GGGTATCG|CCA              (2)
CCCATAGC|GGTTCGA    5'

## Fig.3

## Fig.4